# EUROPEAN PATENT APPLICATION

(11) **EP 4 502 065 A1**
(43) Date of publication of application: **05.02.2025**
(21) Application number: 23780253.3
(22) Date of filing: 24.03.2023
(51) Int. Cl.: C08L 71/02, A61L 31/06, A61L 31/14, C08L 5/16

(54) **METHOD FOR PREPARING SUPRAMOLECULAR HYDROGEL, AND APPLICATION AS BIOMATERIAL**

(30) Priority: 30.03.2022 JP 2022056847
(71) Applicant: Denka Company Limited, Tokyo 103-8338 (JP); National University Corporation Tokyo Medical and Dental University, Tokyo 113-8510 (JP)
(72) Inventor: AOYAMA SEKIYA, Ruriko, Tokyo 103-8338 (JP); WATANABE, Jun, Tokyo 103-8338 (JP); YUI, Nobuhiko, Tokyo 113-8510 (JP); TAMURA, Atsushi, Tokyo 113-8510 (JP); ARISAKA, Yoshinori, Tokyo 113-8510 (JP)
(74) Representative: Müller-Boré & Partner Patentanwälte PartG mbB
(86) International application number: PCT/JP2023/011993
(87) International publication number: WO 2023/190243

(57) **Abstract**

The present invention provides a polyrotaxane having a chemical structure in which an axial molecule represented by Formula (2) is threaded into a cyclodextrin ring in a compound represented by Formula (1). In Formula (1), CD is a cyclodextrin ring, L² is a divalent organic group, L³ is an ethylene group or a propylene group, R^{A} is a hydrogen atom or a C₁₋₆ alkyl group, and m is an integer of 0 to 35. Provided that when L² and N are linked by a double bond, R^{A} is not present. In Formula (2), R is a hydrocarbon group larger than an inner diameter of the cyclodextrin ring, X is a divalent organic group, and a is an integer of 100 to 1000.

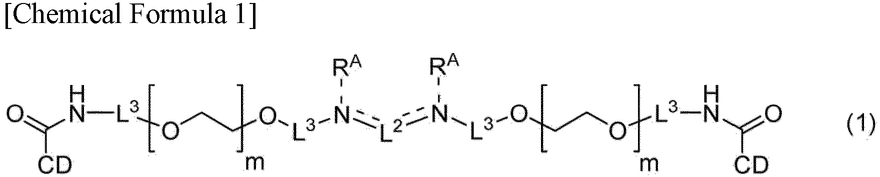

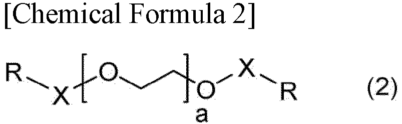

## Description

### Technical Field

The present invention relates to a method for preparing a supramolecular hydrogel and an application as a biomaterial.

### Background Art

In the medical field, a biological tissue is often injured in situations such as closure of a site of a surgical wound, protection of a wound site, and implantation or removal of a medical device. The injured biological tissue begins self-repairing to heal. In this repairing process, other biological tissues existing around the repair site of the biological tissue may be caught and continuously bonded to the repair site. This state is called adhesion. Adhesion may occur between two or more adjacent biological tissues or between an implanted device and a biological tissue adjacent thereto. In order to prevent such adhesion, an adhesion preventing material is used. It is desirable that the material of the adhesion preventing material has biodegradability so that it is not necessary to remove the placed adhesion preventing material.

As the adhesion preventing material, film-shaped, liquid-like, and spray-like adhesion preventing materials have been developed (for example, Patent Literatures 1 to 4), and SEPRAFILM (registered trademark), Adspray (registered trademark), and the like are commercially available. SEPRAFILM is a bioabsorbable film containing sodium hyaluronate and carboxymethylcellulose at a weight ratio of 2 : 1. Adspray is a spray-type adhesion preventing material, and two kinds of solutions extruded from a spraying syringe are uniformly mixed with a gas at the tip of a sprayer and sprayed in an atomized form around a target site. The sprayed solutions quickly gelate to form a physical barrier wall exhibiting an anti-adhesion effect.

### Citation List

### Patent Literature

Patent Literature 1: JP 6403297 B2
Patent Literature 2: JP 6239771 B2
Patent Literature 3: JP 6453412 B2
Patent Literature 4: JP 6562410 B2
Patent Literature 5: JP 2016-89175 A1
Patent Literature 6: WO 2017/191827 A1

### Non Patent Literature

Non Patent Literature 1: Maiko Ozeki et al. PLoS ONE 14 (1): e0211391.

### Summary of Invention

### Technical Problem

However, the film-shaped adhesion preventing material has an advantage of being easy to use without requiring preparation or the like in advance, but has problems in terms of handling such as cracking at the time of use due to low flexibility and stretchability. When a polymer to be a material of a film is produced, heating for a polymerization reaction, use of a catalyst, acidic or alkaline conditions, and the like are required. These are greatly different from the environment used for the living body, and have a technical problem for avoiding adverse effects on the living body.

Therefore, an object of the present invention is to provide a new material that can be produced under simple conditions and can be used as an adhesion preventing material.

### Solution to Problem

The present inventors have focused on the physical properties and biodegradability of a polyrotaxane, and completed the present invention. That is, the present invention provides the following [1] to [16].
[1] A polyrotaxane (polyrotaxane B) having a chemical structure in which an axial molecule represented by Formula (2) is threaded into a cyclodextrin ring in a compound represented by Formula (1): 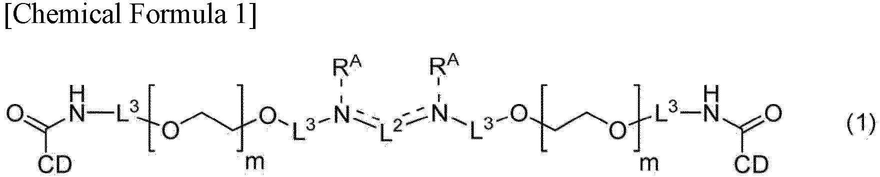
   wherein CD is a cyclodextrin ring, L² is a divalent organic group, L³ is an ethylene group or a propylene group, R^{A} is a hydrogen atom or a C₁₋₆ alkyl group, and m is an integer of 0 to 35, provided that when L² and N are linked by a double bond, R^{A} is not present, 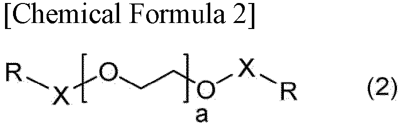
   wherein R is a hydrocarbon group larger than an inner diameter of the cyclodextrin ring, X is a divalent organic group, and a is an integer of 100 to 1000.
[2] A polyrotaxane (polyrotaxane B) having a chemical structure in which an axial molecule represented by Formula (2) is threaded into a cyclodextrin ring in a compound represented by Formula (1'): 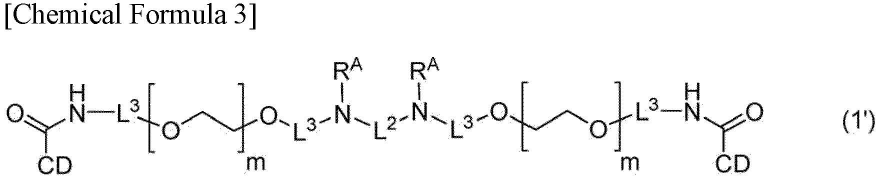
   wherein CD is a cyclodextrin ring, L² is a divalent organic group, L³ is an ethylene group or a propylene group, R^{A} is a hydrogen atom or a C₁₋₆ alkyl group, and m is an integer of 0 to 35, 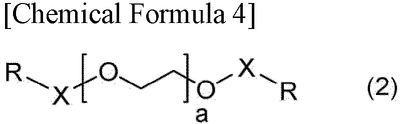
   wherein R is a hydrocarbon group larger than an inner diameter of the cyclodextrin ring, X is a divalent organic group, and a is an integer of 100 to 1000.
[3] The polyrotaxane according to [1] or [2], in which the axial molecule is further threaded into a cyclodextrin ring in a compound represented by Formula (3): 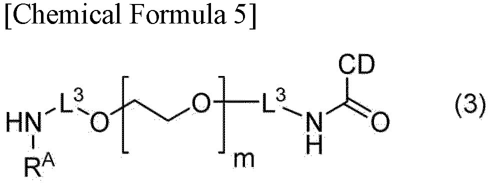 wherein CD is a cyclodextrin ring, L³ is an ethylene group or a propylene group, R^{A} is a hydrogen atom or a C₁₋₆ alkyl group, and m is an integer of 0 to 35.
[4] The polyrotaxane according to any one of [1] to [3], in which the axial molecule is a compound represented by Formula (2a) or Formula (2b): 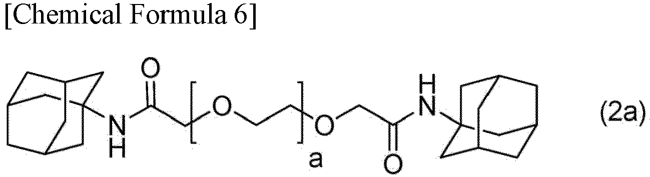
   wherein a is an integer of 100 to 1000, 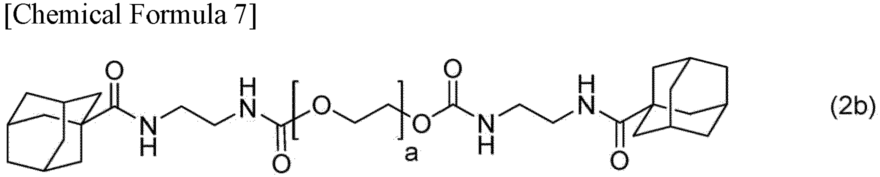
   wherein a is an integer of 100 to 1000.
[5] The polyrotaxane (polyrotaxane B) according to any one of [1] to [4], in which the compound represented by Formula (1) or Formula (1') is a compound represented by Formula (1a-1), (1a-2), or (1a-3): 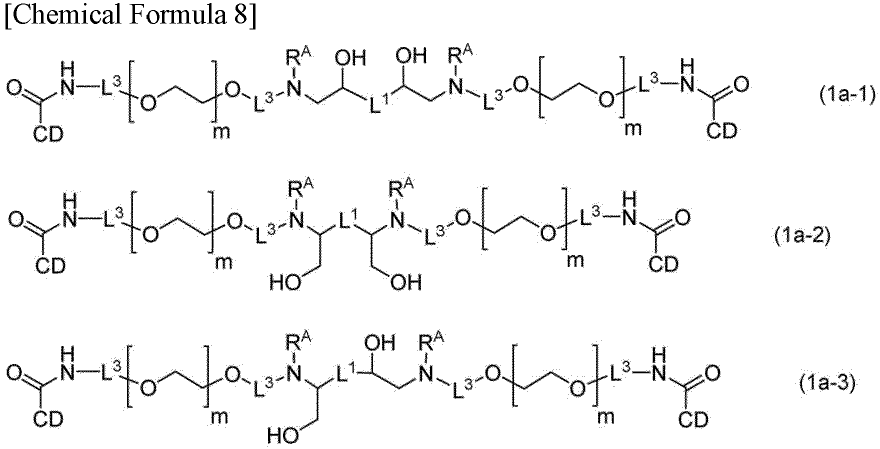 wherein CD is a cyclodextrin ring, L¹ is a divalent organic group, L³ is an ethylene group or a propylene group, R^{A} is a hydrogen atom or a C₁₋₆ alkyl group, and m is an integer of 0 to 35.
[6] The polyrotaxane according to any one of [1] to [5], in which a transmittance of light at a wavelength of 600 nm is 40% or more.
[7] A medical composition comprising the polyrotaxane (polyrotaxane B) according to any one of [1] to [6].
[8] The medical composition according to [7], which is in the form of a hydrogel.
[9] The medical composition according to [7] or [8], which is a material for cell culture, a tissue adhesive, a substrate for tissue regeneration, or an adhesion preventing material.
[10] A method for producing a polyrotaxane (polyrotaxane B) having a chemical structure in which an axial molecule represented by Formula (2) is threaded into a cyclodextrin ring in a compound represented by Formula (1): 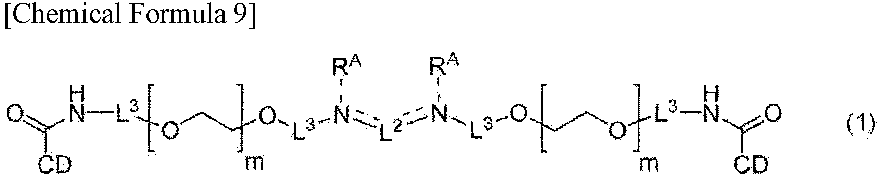
   wherein CD is a cyclodextrin ring, L² is a divalent organic group, L³ is an ethylene group or a propylene group, R^{A} is a hydrogen atom or a C₁₋₆ alkyl group, and m is an integer of 0 to 35, provided that when L² and N are linked by a double bond, R^{A} is not present, 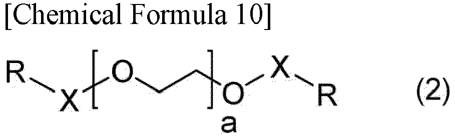
   wherein R is a hydrocarbon group larger than an inner diameter of the cyclodextrin ring, X is a divalent organic group, and a is an integer of 100 to 1000,
   the production method comprising a step of mixing a polyrotaxane (also referred to as "polyrotaxane A") having a chemical structure in which an axial molecule represented by Formula (2) is threaded into each cyclodextrin ring in one or a plurality of compounds represented by Formula (3): 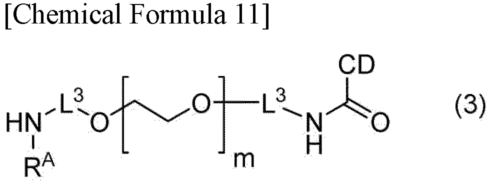
   wherein CD is a cyclodextrin ring, L³ is an ethylene group or a propylene group, R^{A} is a hydrogen atom or a C₁₋₆ alkyl group, and m is an integer of 0 to 35, 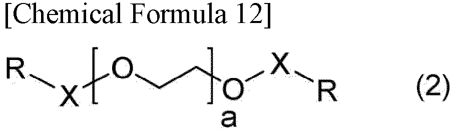
   wherein R is a hydrocarbon group larger than an inner diameter of the cyclodextrin ring, X is a divalent organic group, and a is an integer of 100 to 1000,
   with a crosslinking agent represented by Formula (4):
      [Chemical Formula 13]

      X¹-L¹-X¹ (4)
   wherein L¹ is a divalent organic group, and X¹ is a group capable of reacting with an amino group.
[11] A method for producing a polyrotaxane (polyrotaxane B) having a chemical structure in which an axial molecule represented by Formula (2) is threaded into a cyclodextrin ring in a compound represented by Formula (1'): 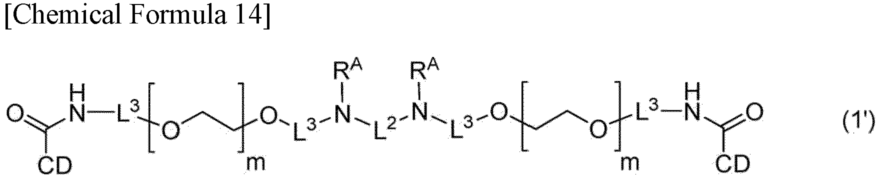
   wherein CD is a cyclodextrin ring, L² is a divalent organic group, L³ is an ethylene group or a propylene group, R^{A} is a hydrogen atom or a C₁₋₆ alkyl group, and m is an integer of 0 to 35, 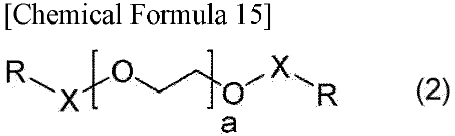
   wherein R is a hydrocarbon group larger than an inner diameter of the cyclodextrin ring, X is a divalent organic group, and a is an integer of 100 to 1000,
   the production method comprising a step of mixing a polyrotaxane (also referred to as "polyrotaxane A") having a chemical structure in which an axial molecule represented by Formula (2) is threaded into each cyclodextrin ring in one or a plurality of compounds represented by Formula (3): 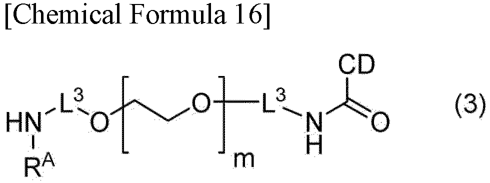
   wherein CD is a cyclodextrin ring, L³ is an ethylene group or a propylene group, R^{A} is a hydrogen atom or a C₁₋₆ alkyl group, and m is an integer of 0 to 35, 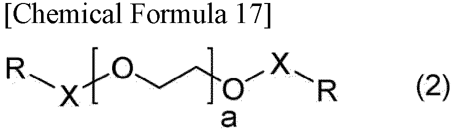
   wherein R is a hydrocarbon group larger than an inner diameter of the cyclodextrin ring, X is a divalent organic group, and a is an integer of 100 to 1000,
   with a crosslinking agent represented by Formula (4):
      [Chemical Formula 18]

      X¹-L¹-X¹ (4)
   wherein L¹ is a divalent organic group, and X¹ is a group capable of reacting with an amino group.
[12] The method according to [10] or [11], in which in the polyrotaxane (polyrotaxane B) having a chemical structure in which an axial molecule represented by Formula (2) is threaded into a cyclodextrin ring in a compound represented by Formula (1) or Formula (1'), the axial molecule is further threaded into a cyclodextrin ring in a compound represented by Formula (3): 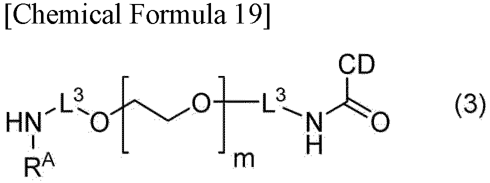 wherein CD is a cyclodextrin ring, L³ is an ethylene group or a propylene group, R^{A} is a hydrogen atom or a C₁₋₆ alkyl group, and m is an integer of 0 to 35.
[13] The production method according to any one of [10] to [12], wherein the step of mixing the polyrotaxane A with the crosslinking agent represented by Formula (4) is performed in the absence of a catalyst.
[14] A kit comprising:
   a polyrotaxane (polyrotaxane A) having a chemical structure in which an axial molecule represented by Formula (2) is threaded into each cyclodextrin ring in one or a plurality of compounds represented by Formula (3); and 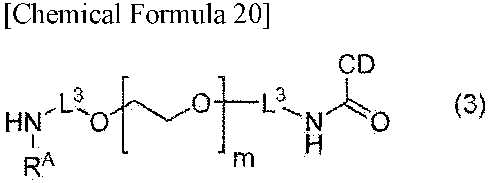
   wherein CD is a cyclodextrin ring, L³ is an ethylene group or a propylene group, R^{A} is a hydrogen atom or a C₁₋₆ alkyl group, and m is an integer of 0 to 35, 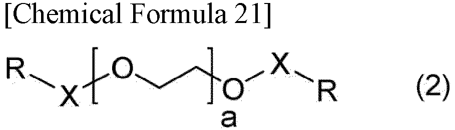
   where R is a hydrocarbon group larger than an inner diameter of the cyclodextrin ring, X is a divalent organic group, and a is an integer of 100 to 1000,
   a crosslinking agent represented by Formula (4):
      [Chemical Formula 22]

      X¹-L¹-X¹ (4)
   wherein L¹ is a divalent organic group, and X¹ is a group capable of reacting with an amino group.
[15] The method for producing a polyrotaxane (polyrotaxane B) according to any one of [10] to [14], in which the compound represented by Formula (1) or Formula (1') is a compound represented by Formula (1a-1), (1a-2), or (1a-3): 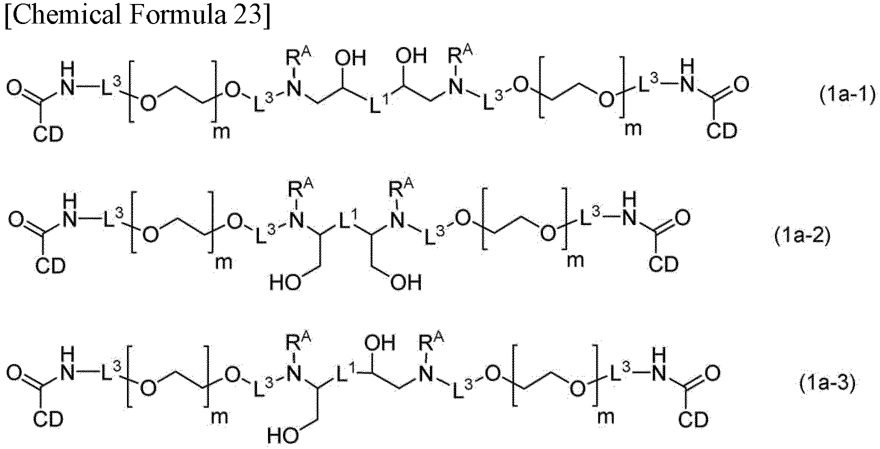 wherein CD is a cyclodextrin ring, L¹ is a divalent organic group, L³ is an ethylene group or a propylene group, R^{A} is a hydrogen atom or a C₁₋₆ alkyl group, and m is an integer of 0 to 35.
[16] A polyrotaxane (polyrotaxane A) having a chemical structure in which an axial molecule represented by Formula (2) is threaded into each cyclodextrin ring in one or a plurality of compounds represented by Formula (3): 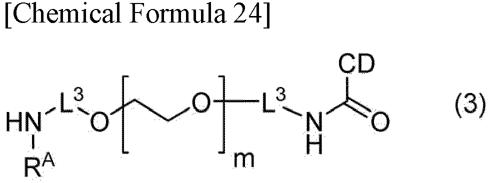
   wherein CD is a cyclodextrin ring, L³ is an ethylene group or a propylene group, R^{A} is a hydrogen atom or a C₁₋₆ alkyl group, and m is an integer of 0 to 35, 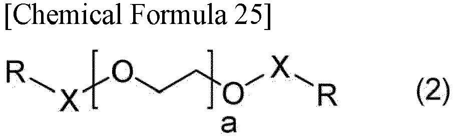
   wherein R is a hydrocarbon group larger than an inner diameter of the cyclodextrin ring, X is a divalent organic group, and a is an integer of 100 to 1000.

### Advantageous Effects of Invention

According to the present invention, it is possible to provide a new material that can be produced under simple conditions and can be used as an adhesion preventing material. The polyrotaxane of the present invention can be easily prepared under mild conditions (for example, temperature ranges from room temperature (0 to 30°C) to body temperature (36 to 37°C)), and neutral (for example, pH of 6 to 9), water-containing, and catalyst-free conditions), and it is also possible to avoid acidic or alkaline conditions, use of an organic solvent, and the like that may adversely affect living bodies. Since the adhesion preventing material comprising the polyrotaxane of the present invention can be used in the form of a transparent hydrogel, followability to an application site is excellent, and the situation of the back side (the degree of adhesion) can be visually recognized while the adhesion preventing material is placed.

### Brief Description of Drawings

FIG. 1 shows a ¹H-NMR spectrum of a polyrotaxane of Production Example 1.
FIG. 2 shows a ¹H-NMR spectrum of a polyrotaxane of Production Example 2B.
FIG. 3 shows appearance photographs of polyrotaxane aqueous solutions of Production Examples 3 and 4.
FIG. 4 shows total transmittance spectra in a visible light region of polyrotaxanes of Production Examples 3 and 4.
FIG. 5 is a graph showing the relationship between the amount of a crosslinking agent used and a gelation time in the case of using a polyrotaxane of Production Example 4.
FIG. 6 shows photographs showing cell adhesiveness in an uncoated polystyrene dish (FIG. 6(a)) and a dish coated with a hydrogel derived from a polyrotaxane of Production Example 2A (FIG. 6(b)).
FIG. 7 shows appearance photographs of hydrogels of Examples 1 to 5 and Comparative Example 1.
FIG. 8 shows appearance photographs of hydrogels of Examples 7 to 10.
FIG. 9 shows total transmittance spectra in a visible light region of hydrogels derived from polyrotaxanes of Production Examples 3 and 4.

### Description of Embodiments

Embodiments of the present invention will be described in detail below.

### [First Embodiment]

A first embodiment of the present invention is a polyrotaxane (polyrotaxane B) having a chemical structure in which an axial molecule represented by Formula (2) is threaded into a cyclodextrin ring in a compound represented by Formula (1).

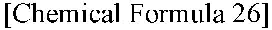

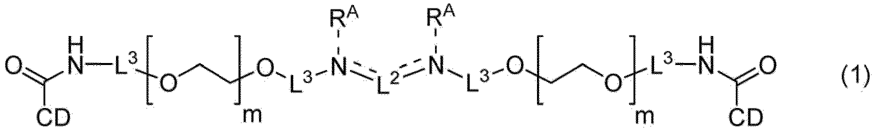

In Formula (1), CD is a cyclodextrin ring, L² is a divalent organic group, L³ is an ethylene group or a propylene group, R^{A} is a hydrogen atom or a C₁₋₆ alkyl group, and m is an integer of 0 to 35. The terminal carbon atom of the L² group is connected to an adjacent nitrogen atom via a single bond or a double bond. When the terminal carbon atom is connected to a nitrogen atom via a single bond, the R^{A} group is a hydrogen atom or a C₁₋₆ alkyl group. When the terminal carbon atom is connected to a nitrogen atom via a double bond, the R^{A} group is not present. The compound represented by Formula (1) has one cyclodextrin ring (CD ring) at each of both ends. Each CD ring may be the same as or different from each other. The R^{A} group is preferably a hydrogen atom. When the R^{A} group is a hydrogen atom, the crosslinking reaction proceeds more smoothly, and the crosslinking density is further increased.

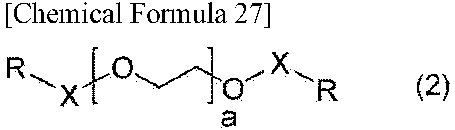

In Formula (2), R is a hydrocarbon group larger than an inner diameter of the cyclodextrin ring, X is a divalent organic group, and a is an integer of 100 to 1000.

The following Formula (1X) is a schematic chemical formula representing the overall structure of an example of the polyrotaxane B. R, X, L², L³, R^{A}, m, and a in Formula (1X) are each as defined in Formula (1) or Formula (2). n is the number of crosslinked CDs threaded into one axial molecule.

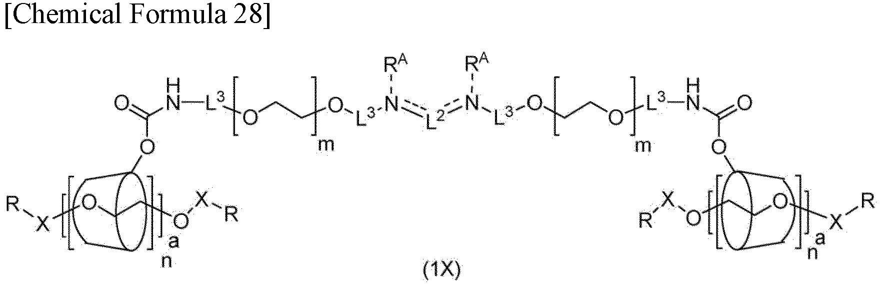

In particular, the following formula is a schematic chemical formula representing a cyclodextrin ring. "OH" refers to a hydroxyl group involved in the reaction among hydroxyl groups in glucose constituting the cyclodextrin for convenience. In the formulae, only one "OH" is shown, but it should not be interpreted restrictively that only one hydroxyl group is present.

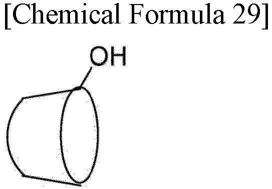

The polyrotaxane B comprises two compounds (axial molecules) represented by Formula (2) and a compound (cyclic molecule) having two modified cyclodextrins (CDs), and has a chemical structure in which one axial molecule is threaded into each cyclodextrin ring. In Formula (1X), each of the axial molecules represented by Formula (2) is threaded into n CDs. CD is bonded to a carbonyl carbon of a urethane bond via an oxygen atom constituting a hydroxyl group thereof. Both ends of the axial molecule are capped with a hydrocarbon group bulkier than the inner diameter of CD to be used so that the CD is not detached.

CD may be any of αCD, βCD, γCD, and combinations thereof. CD is preferably αCD. CD is a compound in which a plurality of glucose are linked by 1,4-glycosyl bonds and linked in a cyclic form. αCD, βCD, and γCD are composed of 6, 7, and 8 glucose, respectively, and have cavity inner diameter of 0.5 to 0.6 nm, 0.7 to 0.8 nm, and 0.9 to 1.0 nm, respectively.

Among the hydroxyl groups contained in the CD, the number of hydroxyl groups to which a side chain (crosslinking part) having another CD linked to a terminal is bonded as shown in Formula (1) (that is, the number of crosslinking parts introduced into one cyclodextrin) may be 1 to 18, preferably 1 to 10, and more preferably 1 to 6 with respect to one cyclodextrin. As the "number of hydroxyl groups to which the crosslinking part is bonded" is larger, it means that side chains having other CDs at many terminals are extended from CD into which one axial molecule is threaded, and respective rotaxane structures (combination of axial molecule and CD threaded thereinto) is more multiply bonded to form a dense network structure.

In CD, an alkyl group having 1 to 3 carbon atoms or an oxyethylene group may be bonded to a hydroxyl group other than the "hydroxyl group to which the crosslinking part is bonded". When these groups are bonded, the water solubility of the polyrotaxane can be further improved.

In Formula (1), m is an integer of 0 to 35, and may be 0 to 33, 0 to 30, 0 to 27, 0 to 24, 0 to 20, 0 to 17, 0 to 13, 0 to 10, 1 to 35, 1 to 33, 1 to 30, 1 to 27, 1 to 24, 1 to 20, 1 to 17, 1 to 14, 1 to 10, 1 to 9, 1 to 8, 1 to 7, 1 to 6, 1 to 5, 1 to 4, 1 to 3, 1 to 2, 2 to 5, 2 to 4, 2 to 3, 3 to 5, or 3 to 4.

In Formula (1), the L² group is a divalent organic group, and may be an aliphatic hydrocarbon group which may contain a hetero atom (for example, an oxygen atom or a nitrogen atom), and may be, for example, an alkylene group or an oxyalkylene group. The L² group is preferably as follows.

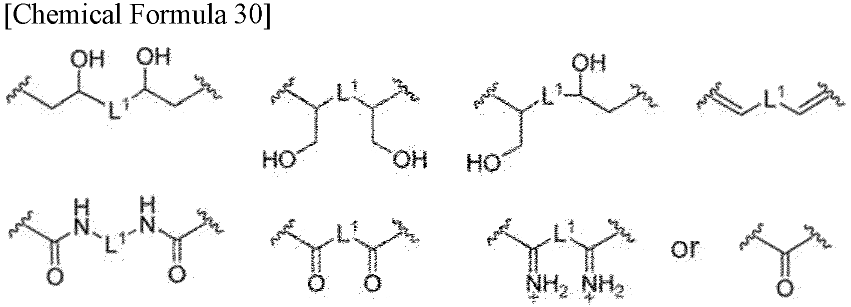

The L¹ group depends on the chemical structure of a crosslinking agent described later. The L¹ group is as defined in Formula (4).

The axial molecule represented by Formula (2) has a polyethylene glycol structure at the center of the molecule and has a -X-R group at the terminal. In Formula (2), the R group is a hydrocarbon group larger than the cavity inner diameter of the CD ring, the X group is a divalent organic group, and cyclodextrin is threaded into the polyethylene glycol structure portion.

The R group may be a hydrocarbon group having such a bulkiness that the CD ring is not separated from the axial molecule. For example, when the CD ring is αCD, the R group has a length of more than 0.6 nm in a direction orthogonal to the axial direction of the axial molecule. Each R group may be the same as or different from each other. The R group is preferably an adamantyl group.

The X group is a divalent organic group linking the polyethylene glycol structure and the R group, and is not particularly limited. Examples of the X group include an alkylene group having 1 to 10 carbon atoms, an alkenylene group having 1 to 20 carbon atoms, and an alkynylene group having 1 to 20 carbon atoms. The alkylene group, the alkenylene group, or the alkynylene group may have an oxo group, an oxy group, or an imino group interposed at any position, or may have any combination thereof. For example, a combination of a carbon atom having an oxo group and an oxy group forms an ester bond, and a combination of a carbon atom having an oxo group and an imino group forms an amide bond. The alkylene group having a plurality of oxy groups is also referred to as an oxyalkylene group, and includes, for example, a polyoxyethylene group having 1 to 10 carbon atoms and a polyoxypropylene group having 1 to 10 carbon atoms. Specific examples of the organic group include alkylene groups such as a methylene group, an ethylene group, a propylene group, a butylene group, a pentylene group, a hexylene group, a heptylene group, an octylene group, a nonanylene group, and a decylene group; alkenylene groups such as a propynylene group, a butenylene group, a pentenylene group, a hexenylene group, a heptenylene group, an octenylene group, a nonenylene group, and a decenylene group; and alkynylene groups such as a propargyl group, a butynylene group, a pentynylene group, a hexynylene group, a heptynylene group, an octynylene group, a nonylene group, and a decynylene group. An organic group having an oxo group, an oxy group, or an imino group preferably forms an ester bond, a carbonate bond, or a urethane bond together with an oxygen atom derived from the hydroxyl group of the cyclodextrin. Examples of the organic group having an oxo group, an oxy group, or an imino group include a carbonylmethylene group (-C(=O)CH₂-), a methylenecarbonyl group (-CH₂C(=O)-), a carbonylmethylene group (-C(=O)CH₂-), a methylenecarbonylamino group (-CH₂C(=O)NH-), a carbonylaminoethylene group (-C(=O)NHCH₂-), and a carbonylaminoethyleneoxyethylene group (-C(=O)NHCH₂CH₂OCH₂CH₂-). The X group preferably has an amide bond, a urethane bond, or a urea bond, is advantageous for introducing the R group into a polyethylene glycol structure, and is more excellent in biodegradability. When the X group has a disulfide bond (represented by Formula (A)), an o-nitrobenzyl structure (represented by Formula (B)), a hydrazone structure (represented by Formula (C)), or a ketal structure (represented by Formula (D)) shown below, the biodegradability is further excellent.

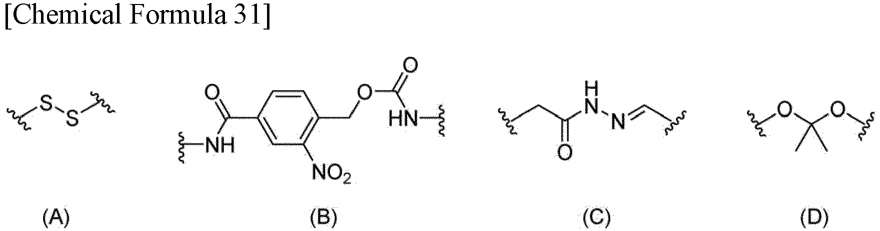

The number of threaded cyclodextrins per axial molecule can be independently determined and need not be uniquely determined by the molecular length of the polyethylene glycol structure. Stoichiometrically, since CD can include two units of ethylene glycol, which is a repeating unit of polyethylene glycol, the number of threaded cyclodextrins has an upper limit depending on the molecular weight of the axial molecule to be used. For example, the number of threaded CDs per polyethylene glycol molecule having a number average molecular weight of 20000 may be 3 to 220, and is preferably 5 to 150, more preferably 5 to 120, and particularly preferably 5 to 100. However, the length (total length when the polyrotaxane has a plurality of CDs) of the axial molecule of the cyclodextrin in the main chain direction does not exceed the molecular length of polyethylene glycol.

The polyethylene glycol structure in Formula (2) may include ethylene glycol as a monomer unit and have a molecular length capable of threading at least one cyclodextrin. The number of ethylene glycol units forming the polyethylene glycol structure may be 60 to 1000, and is more preferably 65 to 950, 80 to 900, 90 to 900, 100 to 900, 110 to 900, 130 to 880, 150 to 850, 180 to 830, 200 to 800, 230 to 770, 250 to 750, 250 to 700, 270 to 680, or 350 to 650.

Specific examples of the axial molecule represented by Formula (2) include a compound represented by Formula (2a) or Formula (2b).

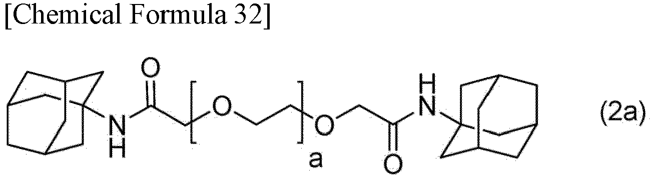

In the formula, a is an integer of 100 to 1000.

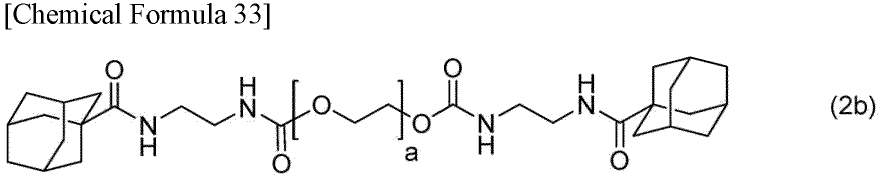

In the formula, a is an integer of 100 to 1000.

In an embodiment, the polyrotaxane B preferably further comprises a compound represented by Formula (3). In this case, the compound represented by Formula (3) is included such that the axial molecule is threaded into the CD ring. The compound represented by Formula (3) contained in the polyrotaxane B has a side chain having a terminal amino group, and can exhibit an effect of further improving cell adhesiveness and tissue adhesiveness. When CD is αCD, the number of side chains having a terminal amino group may be 1 to 17 and is preferably 1 to 8 and more preferably 1 to 6 per CD.

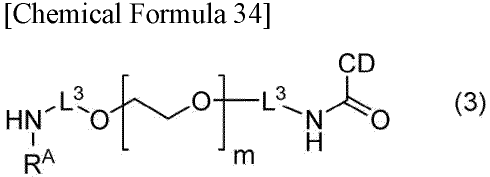

In Formula (3), CD is a cyclodextrin ring, L³ is an ethylene group or a propylene group, R^{A} is a hydrogen atom or a C₁₋₆ alkyl group, and m is an integer of 0 to 35.

When the polyrotaxane B further comprises the compound represented by Formula (3), the polyrotaxane B comprises both the compound represented by Formula (1) and the compound represented by Formula (3), and is represented by, for example, Formula (1Xa).

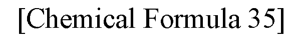

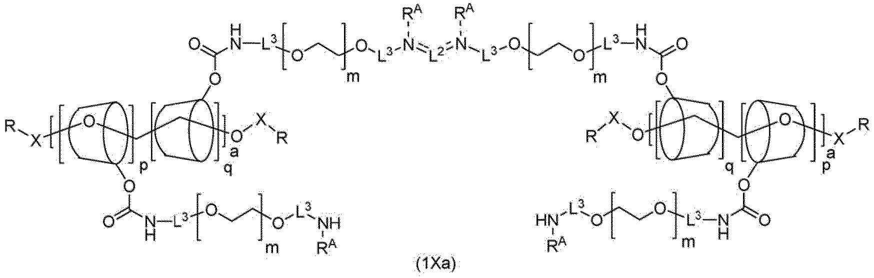

R, X, L², L³, R^{A}, m, and a in Formula (1Xa) are each as defined in Formula (1X). The terminal carbon atom of the L² group is connected to an adjacent nitrogen atom via a single bond or a double bond. When the terminal carbon atom is connected to a nitrogen atom via a single bond, the R^{A} group is a hydrogen atom or a C₁₋₆ alkyl group. When the terminal carbon atom is connected to a nitrogen atom via a double bond, the R^{A} group is not present.

The compound represented by Formula (1) in the polyrotaxane B is preferably the compound represented by Formula (1a-1). In an embodiment, the compound represented by Formula (1) further comprises a compound represented by Formula (1a-2) or (1a-3). In Formulas (1a-1), (1a-2), and (1a-3), CD is a cyclodextrin ring, L¹ is a divalent organic group, L³ is an ethylene group or a propylene group, R^{A} is a hydrogen atom or a C₁₋₆ alkyl group, and m is an integer of 0 to 35. The number of threaded CDs in the polyrotaxane B is the total value of the number of threaded CDs contained in each compound represented by Formula (1) or Formula (3).

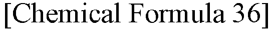

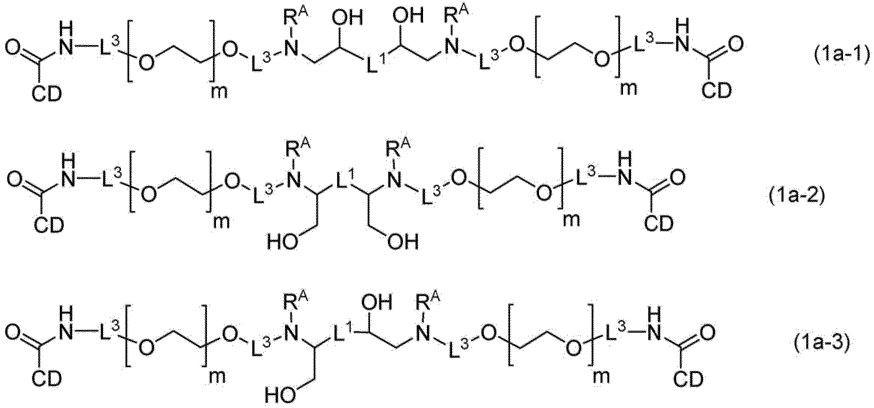

In each formula, CD is a cyclodextrin ring, L¹ is a divalent organic group, L³ is an ethylene group or a propylene group, R^{A} is a hydrogen atom or a C₁₋₆ alkyl group, and m is an integer of 0 to 35.

In another embodiment, the compound represented by Formula (1) may be a compound represented by Formula (1b), (1c), (1d), (1e), or (1f). in the formula, CD is a cyclodextrin ring, L¹ is a divalent organic group, L³ is an ethylene group or a propylene group, R^{A} is a hydrogen atom or a C₁₋₆ alkyl group, and m is an integer of 0 to 35. The partial structure represented by L² in Formula (1) corresponds to the chemical structure of the compound represented by Formula (4). In Formula (1b), the R^{A} group is not present.

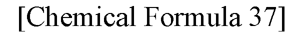

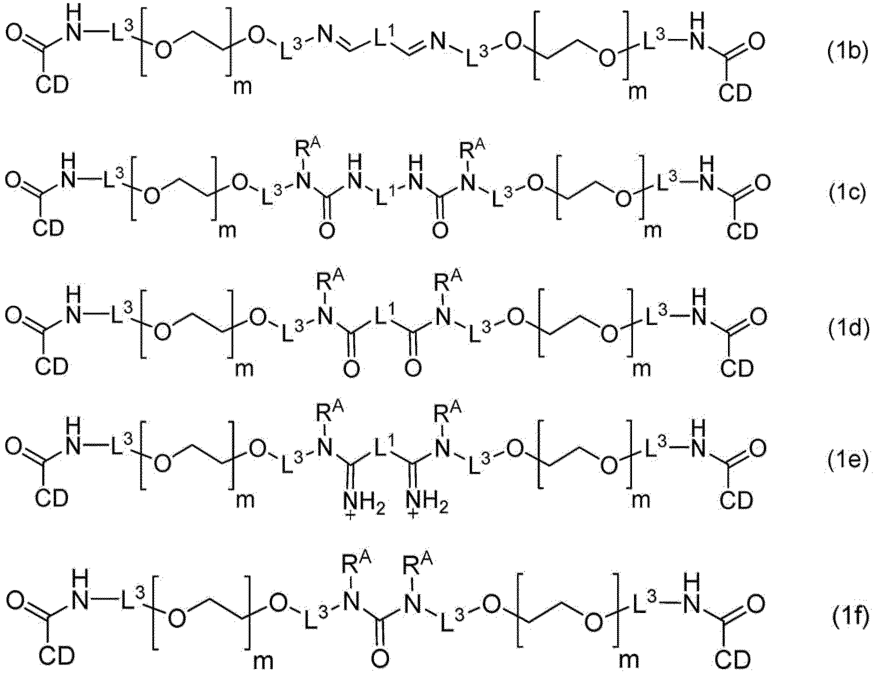

In each formula, CD is a cyclodextrin ring, L¹ is a divalent organic group, L³ is an ethylene group or a propylene group, R^{A} is a hydrogen atom or a C₁₋₆ alkyl group, and m is an integer of 0 to 35.

In the polyrotaxane B, as the length of the axial molecule, the number of threaded CDs per axial molecule, and/or the number of "modified hydroxyl groups" per CD increases, or as the molar ratio of the compound represented by Formula (1) to the axial molecule represented by Formula (2) increases, the network structure formed by linking the compound represented by Formula (1) and the axial molecule represented by Formula (2) as a whole becomes denser. More specifically, when the separate axial molecule represented by Formula (2) is threaded into two CD rings present at both terminals of the compound represented by Formula (1), the two axial molecules represented by Formula (2) are linked. A plurality of compounds represented by Formula (1) are threaded into one axial molecule, another CD ring is present at each terminal, and further another axial molecule is threaded. By linking the plurality of axial molecules in this manner, a network structure is formed as a whole. When the network structure is sufficiently dense, the hydrogel is less likely to be physically brittle and easier to handle. When the network structure is too dense, the molecular mobility of CD may be reduced, and the mechanical properties or water content of the hydrogel may be reduced. When the length of the axial molecule, the number of threaded CDs per axial molecule, and the number of "modified hydroxyl groups" per CD are in the above ranges, the denseness of the network structure can be the degree suitable for use as an adhesion preventing material. As the molar ratio of the compound represented by Formula (1) to the axial molecule represented by Formula (2) is higher, the number of crosslinked structures increases, and a denser network structure can be obtained.

A preferred polyrotaxane B is a polyrotaxane (polyrotaxane B) having a chemical structure in which an axial molecule represented by Formula (2) is threaded into a cyclodextrin ring in a compound represented by Formula (1').

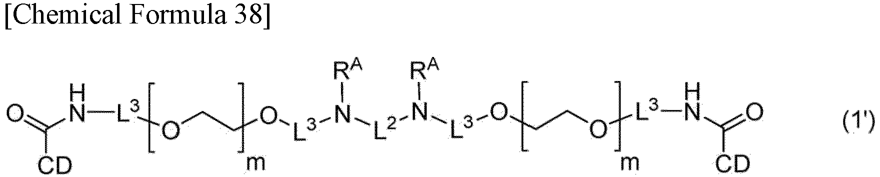

In Formula (1'), CD is a cyclodextrin ring, L² is a divalent organic group, L³ is an ethylene group or a propylene group, R^{A} is a hydrogen atom or a C₁₋₆ alkyl group, and m is an integer of 0 to 35.

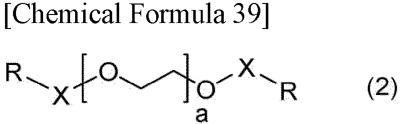

In Formula (2), R is a hydrocarbon group larger than an inner diameter of the cyclodextrin ring, X is a divalent organic group, and a is an integer of 100 to 1000.

### [Second Embodiment]

A second embodiment of the present invention is a medical composition comprising the polyrotaxane B according to the first embodiment. The medical composition may be in any form that can be used in the medical field, and for example, a hydrogel, a powder, a sheet, or a film is preferable. The medical composition may be a material for cell culture, a tissue adhesive, a substrate for tissue regeneration, or an adhesion preventing material. Examples of the material for cell culture or the substrate for tissue regeneration include a coating agent for a polystyrene dish for cell culture and a cell culture substrate (for example, a coated polystyrene dish), focusing on low cytotoxicity due to polyrotaxane, cell adhesiveness, and the like. The tissue adhesive is, for example, an adhesive for adhering tissues to each other, focusing on adhesion of the polyrotaxane according to the first embodiment to cells or tissues. The medical composition according to the present embodiment is particularly suitable for use as an adhesion preventing material.

When the medical composition according to the present embodiment has excellent translucency, microscopic observation can be facilitated when cells are cultured on a cell culture substrate. The polyrotaxane B can be produced in a relatively short time by mixing the polyrotaxane A and a crosslinking agent under mild conditions (temperature ranges from room temperature (0 to 30°C) to body temperature (36 to 37°C), neutral (for example, pH of 6 to 9), water-containing, and catalyst-free), and can be used as an injectable gel that is hydrogelated at an affected part. The injectable gel can also be hydrogelated by previously encapsulating a necessary drug or an active ingredient (growth factor or the like), and is more suitable for use as a cell culture substrate, a tissue adhesive, a regenerated medical substrate, or an adhesion preventing material.

In the present specification, the "anti-adhesion effect" refers to preventing adhesion of a biological tissue (for example, adhesion between organs or adhesion between an organ and peritoneum) without substantially imparting stimulation or adverse effects to the biological tissue. More specifically, it means that the material does not generate or elute a substance harmful to the biological tissue, and the biological tissue in contact with the material determines that the material is a foreign substance and does not show a defense reaction such as inflammation or blood coagulation, or the degree thereof is slight.

When the X group of the axial molecule has a disulfide bond, an o-nitrobenzyl structure, a hydrazone structure, or a ketal structure, the polyrotaxane B is more excellent in biodegradability, and thus the adhesion preventing material can also exhibit biodegradability. Such an adhesion preventing material may suture the skin while being placed on the biological tissue. The placed adhesion preventing material is gradually decomposed and disappears while preventing adhesion of the biological tissue after surgery. Since the adhesion preventing material according to the present embodiment is a hydrogel having translucency, the attachment site is easily visually recognized, and the degree of adhesion can be visually recognized without removing the adhesion preventing material.

The adhesion preventing material according to the present embodiment can be used by being placed on the biological tissue exposed at the time of incision or wound. The site where the adhesion preventing material is used is not particularly limited, and the adhesion preventing material can be used for a site of a surgical wound and a wound site, and can be used for, for example, an arm, a chest, an abdomen, a waist, a back, buttocks, a thigh, a leg, and the like. The size of the adhesion preventing material may be adjusted according to the site to which the adhesion preventing material is applied.

The medical composition according to the present embodiment can be prepared in the form of a hydrogel at need by mixing the polyrotaxane A and the crosslinking agent represented by Formula (4). In about 3 to 30 minutes after mixing, gelation proceeds and the fluidity decreases. Therefore, the polyrotaxane B can also be formed by applying and mixing two solutions on a biological tissue, and thus it is also suitable for use in laparoscopic surgery. Since it takes about 3 to 30 minutes for the hydrogel to lose fluidity, an adhesion preventing material, which fits the shape of a biological tissue, can be obtained. The adhesion preventing material prepared by gelation in advance may be used in a method of placing the adhesion preventing material in a biological tissue. The medical composition may be a lump, a sheet, or a powder obtained by mixing the polyrotaxane A and the crosslinking agent represented by Formula (4), gelating the mixture, and then performing freeze-drying. The medical composition having such a shape can also be used by a method of placing or spraying the medical composition at a desired position (for example, affected part), and then applying physiological saline to return the medical composition to a gel state. The medical composition can be used in a wider range of applications by mixing a necessary drug or an active ingredient (growth factor or the like) in a medical composition in a lump or powder form, or by wrapping a necessary drug or an active ingredient (growth factor or the like) in a sheet-shaped medical composition and then applying physiological saline.

The medical composition according to the present embodiment comprises the polyrotaxane B according to the first embodiment, and may further comprise an optional component as necessary. Examples of the optional component include an antioxidant, a weathering stabilizer, a heat stabilizer, a lubricant, a crystal nucleating agent, an ultraviolet absorber, a colorant, and an antibacterial agent. The content of the optional component may be 0.5 to 10 mass% with respect to the total mass of the medical composition. When the medical composition is a lump, a sheet, or a powder, the content of the polyrotaxane B may be 30 mass% or more, and may be 50 to 99.5 mass%, 50 to 95 mass%, 55 to 90 mass%, 60 to 90 mass%, 65 to 90 mass%, 70 to 90 mass%, 75 to 90 mass%, 80 to 90 mass%, 85 to 90 mass%, 60 to 85 mass%, 60 to 80 mass%, 65 to 80 mass%, 65 to 75 mass%, or 65 to 70 mass%, with respect to the total mass of the medical composition. When the medical composition is a hydrogel, the content of the polyrotaxane B may be 0.5 to 50 mass%, 2 to 50 mass%, 2 to 45 mass%, 5 to 45 mass%, 5 to 40 mass%, 7 to 40 mass%, 7 to 35 mass%, or 10 to 35 mass%, with respect to the total mass of the medical composition.

The medical composition according to the present embodiment may be in the form of a hydrogel. In the present specification, the "hydrogel" means a swollen body of a polymer substance having a three-dimensional structure insoluble in water. The hydrogel is substantially composed of the polyrotaxane B according to the first embodiment and water. The polyrotaxane B according to the first embodiment can be produced according to a production method described below, and since the reaction between the polyrotaxane A and the crosslinking agent represented by Formula (4) proceeds in a short time even under mild conditions (for example, temperature ranges from room temperature (0 to 30°C) to body temperature (36 to 37°C), and neutral (for example, pH of 6 to 9), water-containing, and catalyst-free conditions), the polyrotaxane B can be produced in the form of a hydrogel by mixing aqueous solutions comprising both raw materials. As the polyrotaxane concentration is higher and as the amount of the crosslinking agent used is larger, the storage elastic modulus of the hydrogel to be obtained is increased, and the degree of swelling in water tends to be further reduced.

The medical composition according to the present embodiment is preferably translucent. In the present specification, "translucent" means that a transmittance of 15% or more is exhibited over the entire visible light region (wavelength: 380 to 780 nm) at an optical path length of 10 mm. That is, "translucent" can also be understood as a transmittance of 15% or more/10 mm. The medical composition according to the present embodiment preferably exhibits a transmittance of 18% or more, 20% or more, 25% or more, 30% or more, 35% or more, or 40% or more in the entire visible light region. In particular, the medical composition according to the present embodiment preferably exhibits a transmittance of 40% or more, 45% or more, 50% or more, 55% or more, 60% or more, 65% or more, or 70% or more at a wavelength of 600 nm.

The transmittance of light can be measured under the following conditions, for example. As a measurement method, a general light transmittance measurement method can be referred to, and examples described later may be referred to.

### (Measurement Conditions)

Apparatus: Hitachi spectrophotometer (product name: U-2910)
Measurement mode: wavelength scan
Data mode: Abs mode
Measurement wavelength: 210 nm to 830 nm
Scan speed: 400 nm/min
Cell: polystyrene cell (manufactured by Fisher Scientific K.K.)
Cell length: 10 mm
Baseline: using MilliQ water

### [Third Embodiment]

A third embodiment of the present invention is a method for producing a polyrotaxane (polyrotaxane B) having a chemical structure in which an axial molecule represented by Formula (2) is threaded into each cyclodextrin ring in one or a plurality of compounds represented by Formula (1).

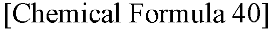

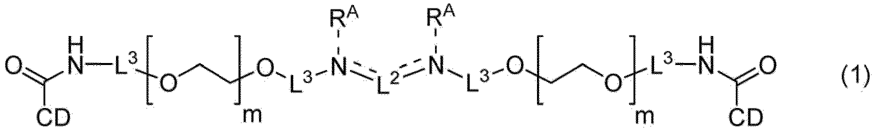

In the formula, CD is a cyclodextrin ring, L² is a divalent organic group, L³ is an ethylene group or a propylene group, R^{A} is a hydrogen atom or a C₁₋₆ alkyl group, and m is an integer of 0 to 35, provided that when L² and N are linked by a double bond, R^{A} is not present.

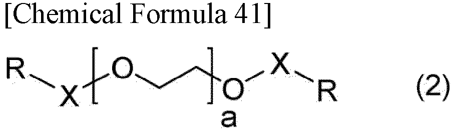

In the formula, R is a hydrocarbon group larger than an inner diameter of the cyclodextrin ring, X is a divalent organic group, and a is an integer of 100 to 1000.

The method for producing a polyrotaxane B according to the present embodiment comprises a step of mixing a polyrotaxane (polyrotaxane A) having a chemical structure in which an axial molecule represented by Formula (2) is threaded into each cyclodextrin ring in one or a plurality of compounds represented by Formula (3): 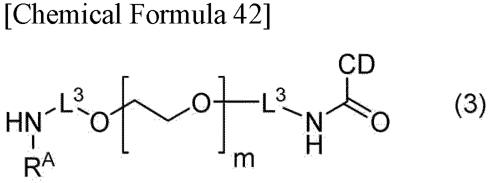
wherein CD is a cyclodextrin ring, L³ is an ethylene group or a propylene group, R^{A} is a hydrogen atom or a C₁₋₆ alkyl group, and m is an integer of 0 to 35, 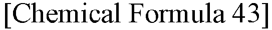 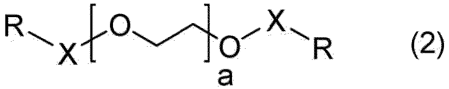
where R is a hydrocarbon group larger than an inner diameter of the cyclodextrin ring, X is a divalent organic group, and a is an integer of 100 to 1000,
with a crosslinking agent represented by Formula (4):
   [Chemical Formula 44]

   X¹-L¹-X¹ (4)
wherein L¹ is a divalent organic group, and X¹ is a group capable of reacting with an amino group.

In this step, by mixing the polyrotaxane A and the crosslinking agent represented by Formula (4), two molecules of the polyrotaxane A are reacted with the crosslinking agent represented by Formula (4) to produce the polyrotaxane B. The reaction in this step does not require the presence of a catalyst and proceeds even under mild conditions (for example, temperature ranges from room temperature (0 to 30°C) to body temperature (36 to 37°C), and neutral (for example, pH of 6 to 9), water-containing, and catalyst-free conditions). In the polyrotaxane A, when the R^{A} group is a hydrogen atom, the crosslinking reaction proceeds more smoothly, and the polyrotaxane B having a higher crosslinking density is obtained. Since no catalyst is required for the crosslinking reaction, it can be used for the preparation at need and does not require purification before use. There is also an advantage that the catalyst does not remain in the living body even when the crosslinking reaction is performed on a target biological tissue.

The following formula is a chemical formula schematically representing this step. In this step, a polyrotaxane A represented by Formula (3X) and the crosslinking agent represented by Formula (4) are bonded to produce the polyrotaxane B.

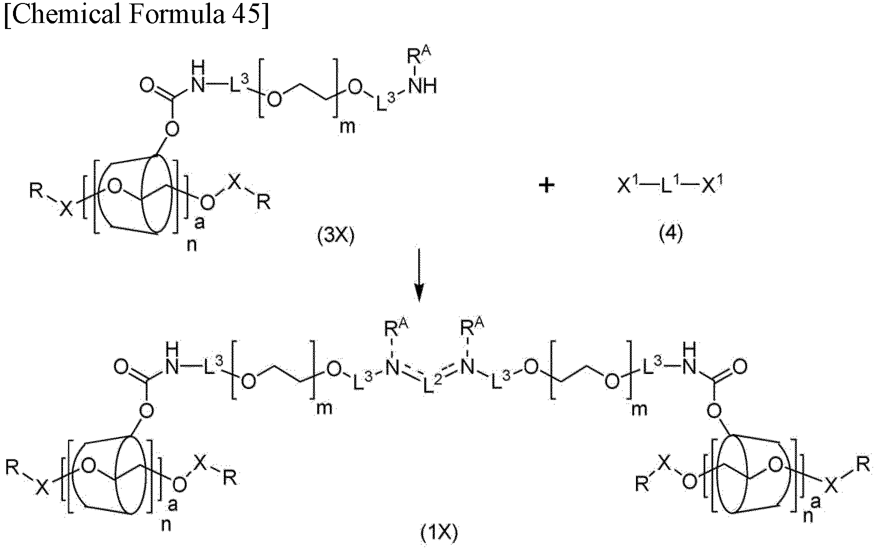

In each formula, R is a hydrocarbon group larger than an inner diameter of the cyclodextrin ring, X is a divalent organic group, L³ is an ethylene group or a propylene group, a is an integer of 100 to 1000, R^{A} is a hydrogen atom or a C₁₋₆ alkyl group, n is an integer of 100 to 1000, m is an integer of 0 to 35, L¹ is a divalent organic group, X¹ is a group capable of reacting with an amino group. Provided that when L² and N are linked by a double bond, R^{A} is not present. L¹ is as defined in Formula (4). L² is as follows.

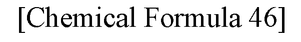

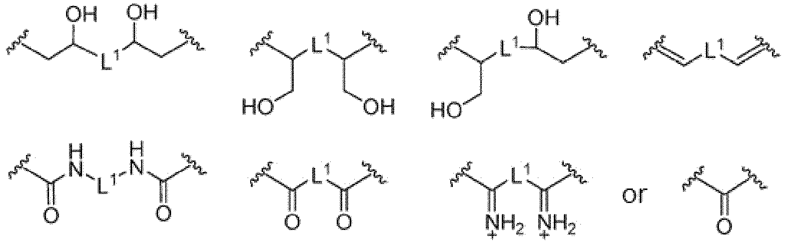

This step may be performed in a solvent or may be performed in the absence of a solvent. It is preferable to mix the polyrotaxane A and the crosslinking agent represented by Formula (4) in the presence of a solvent. The solvent is not limited as long as it does not suppress or inhibit the desired reaction, and can be appropriately selected according to the application. A preferred solvent is water. Practically, it is most convenient to prepare a solution comprising the polyrotaxane A and a solution comprising the crosslinking agent represented by Formula (4), and mix these two solutions at the time of use. The amount of the solvent is not limited as long as the reaction between the polyrotaxane A and the crosslinking agent proceeds at a practical rate. The amount of the solvent may be, for example, 100000 to 10000000 mL with respect to 1 mol of the amount of the polyrotaxane A used, or may be 500000 to 100000000 mL with respect to 1 mol of the amount of the crosslinking agent used.

In this step, the pH of the reaction system may be neutral, and the pH is, for example, 6 to 9 and may be preferably 6 to 8, 6.5 to 8, 6.5 to 7.5, 6.5 to 7, 6 to 7.5, or 6 to 7.

The polyrotaxane A is a polyrotaxane having a chemical structure in which an axial molecule represented by Formula (2) is threaded into each cyclodextrin ring in one or a plurality of compounds represented by Formula (3).

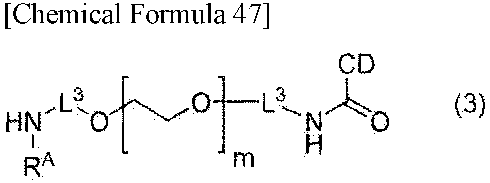

In the formula, CD is a cyclodextrin ring, L³ is an ethylene group or a propylene group, R^{A} is a hydrogen atom or a C₁₋₆ alkyl group, and m is an integer of 0 to 35.

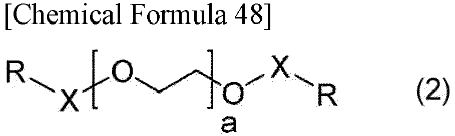

In the formula, R is a hydrocarbon group larger than an inner diameter of the cyclodextrin ring, X is a divalent organic group, and a is an integer of 100 to 1000.

CD is the same as the definition in Formula (1), and may be any of αCD, βCD, γCD, and combinations thereof. CD is preferably αCD. CD is a compound in which a plurality of glucose are linked by 1,4-glycosyl bonds and linked in a cyclic form. αCD, βCD, and γCD are composed of 6, 7, and 8 glucose, respectively, and have cavity inner diameter of 0.5 to 0.6 nm, 0.7 to 0.8 nm, and 0.9 to 1.0 nm, respectively.

Among the hydroxyl groups contained in the CD, the number of hydroxyl groups to which a side chain having another CD linked to a terminal is bonded as shown in Formula (3) (that is, the number of side chains introduced into one cyclodextrin) may be 1 to 18, preferably 1 to 10, and more preferably 1 to 6 with respect to one cyclodextrin. As the "number of hydroxyl groups to which the side chain is bonded" is larger, it means that the side chain having other CD at many terminals extends from the CD into which one axial molecule is threaded.

In CD, an alkyl group having 1 to 3 carbon atoms or an oxyethylene group may be bonded to a hydroxyl group other than the "hydroxyl group to which the side chain is bonded". When these groups are bonded, the water solubility of the polyrotaxane can be further improved.

In Formula (3), m is an integer of 0 to 35, and may be 0 to 33, 0 to 30, 0 to 27, 0 to 24, 0 to 20, 0 to 17, 0 to 13, 0 to 10, 1 to 35, 1 to 33, 1 to 30, 1 to 27, 1 to 24, 1 to 20, 1 to 17, 1 to 14, 1 to 10, 1 to 9, 1 to 8, 1 to 7, 1 to 6, 1 to 5, 1 to 4, 1 to 3, 1 to 2, 2 to 5, 2 to 4, 2 to 3, 3 to 5, or 3 to 4.

The axial molecule represented by Formula (2) has a polyethylene glycol structure at the center of the molecule and has a -X-R group at the terminal. In Formula (2), the R group is a hydrocarbon group larger than the cavity inner diameter of the CD ring, the X group is a divalent organic group, and cyclodextrin is threaded into the polyethylene glycol structure portion.

The R group may be a hydrocarbon group having such a bulkiness that the CD ring is not separated from the axial molecule. For example, when the CD ring is αCD, the R group has a length of more than 0.6 nm in a direction orthogonal to the axial direction of the axial molecule. Each R group may be the same as or different from each other. The R group is preferably an adamantyl group.

The X group is a divalent organic group linking the polyethylene glycol structure and the R group, and is not particularly limited. Examples of the X group include an alkylene group having 1 to 10 carbon atoms, an alkenylene group having 1 to 20 carbon atoms, and an alkynylene group having 1 to 20 carbon atoms. The alkylene group, the alkenylene group, or the alkynylene group may have an oxo group, an oxy group, or an imino group interposed at any position, or may have any combination thereof. For example, a combination of a carbon atom having an oxo group and an oxy group forms an ester bond, and a combination of a carbon atom having an oxo group and an imino group forms an amide bond. The alkylene group having a plurality of oxy groups is also referred to as an oxyalkylene group, and includes, for example, a polyoxyethylene group having 1 to 10 carbon atoms and a polyoxypropylene group having 1 to 10 carbon atoms. Specific examples of the organic group include alkylene groups such as a methylene group, an ethylene group, a propylene group, a butylene group, a pentylene group, a hexylene group, a heptylene group, an octylene group, a nonanylene group, and a decylene group; alkenylene groups such as a propynylene group, a butenylene group, a pentenylene group, a hexenylene group, a heptenylene group, an octenylene group, a nonenylene group, and a decenylene group; and alkynylene groups such as a propargyl group, a butynylene group, a pentynylene group, a hexynylene group, a heptynylene group, an octynylene group, a nonylene group, and a decynylene group. An organic group having an oxo group, an oxy group, or an imino group preferably forms an ester bond, a carbonate bond, or a urethane bond together with an oxygen atom derived from the hydroxyl group of the cyclodextrin. Examples of the organic group having an oxo group, an oxy group, or an imino group include a carbonylmethylene group (-C(=O)CH₂-), a methylenecarbonyl group (-CH₂C(=O)-), a carbonylmethylene group (-C(=O)CH₂-), a methylenecarbonylamino group (-CH₂C(=O)NH-), a carbonylaminoethylene group (-C(=O)NHCH₂-), and a carbonylaminoethyleneoxyethylene group (-C(=O)NHCH₂CH₂OCH₂CH₂-). The X group preferably has an amide bond, a urethane bond, or a urea bond, and it is advantageous for introducing the R group into a polyethylene glycol structure, and is more excellent in biodegradability. When the X group has a disulfide bond (represented by Formula (A)), an o-nitrobenzyl structure (represented by Formula (B)), a hydrazone structure (represented by Formula (C)), or a ketal structure (represented by Formula (D)) shown below, the biodegradability is further excellent.

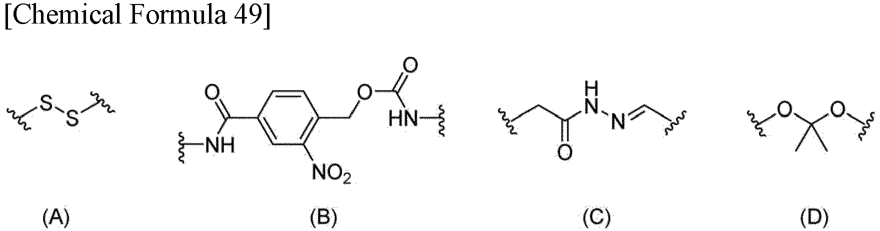

The number of threaded cyclodextrins per axial molecule can be independently determined and need not be uniquely determined by the molecular length of the polyethylene glycol structure. Stoichiometrically, since CD can include two units of ethylene glycol, which is a repeating unit of polyethylene glycol, the number of threaded cyclodextrins has an upper limit depending on the molecular weight of the axial molecule to be used. For example, the number n of threaded CDs per polyethylene glycol molecule having a number average molecular weight of 20000 may be 3 to 220, and is preferably 5 to 150, more preferably 5 to 120, and particularly preferably 5 to 100. However, the length (total length when the polyrotaxane has a plurality of CDs) of the axial molecule of the cyclodextrin in the main chain direction does not exceed the molecular length of polyethylene glycol.

The polyethylene glycol structure in Formula (2) may include ethylene glycol as a monomer unit and have a molecular length capable of threading at least one cyclodextrin. The number of ethylene glycol units forming the polyethylene glycol structure may be 60 to 1000, and is more preferably 65 to 950, 80 to 900, 90 to 900, 100 to 900, 110 to 900, 130 to 880, 150 to 850, 180 to 830, 200 to 800, 230 to 770, 250 to 750, 250 to 700, 270 to 680, or 350 to 650.

Specific examples of the axial molecule represented by Formula (2) include a compound represented by Formula (2a) or Formula (2b).

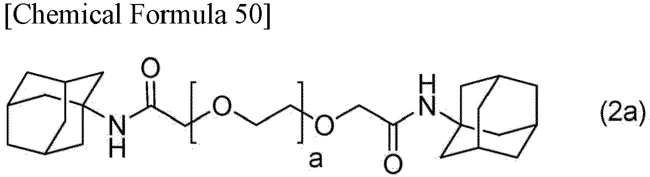

In the formula, a is an integer of 100 to 1000.

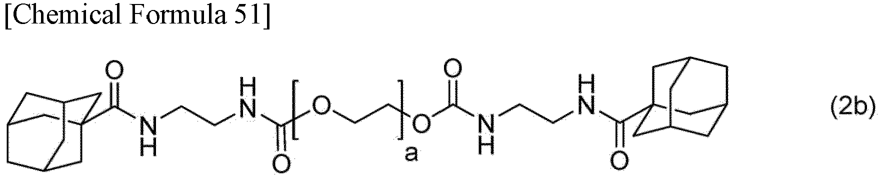

In the formula, a is an integer of 100 to 1000.

The crosslinking agent is a compound represented by Formula (4). The L¹ group is a divalent organic group, and is preferably a chain or branched alkane and more preferably C₁₋₁₀ alkane. The L¹ group may be an aliphatic hydrocarbon group which may comprise a hetero atom (for example, an oxygen atom or a nitrogen atom), and may be, for example, a carbonyl group, an alkylene group, or an oxyalkylene group. The X¹ group may be a group capable of reacting with a primary amino group or a secondary amino group, and examples thereof include an oxiranyl group, a formyl group, an isocyanate group, a succinimidyloxycarbonyl group, a 3-sulfosuccinimidyloxycarbonyl group, and a carboxyimidate group. Among them, one or more selected from the group consisting of an oxiranyl group, a formyl group, and an isocyanate group are preferable because no byproduct is generated by the reaction of the amino group. A more preferred crosslinking agent is a compound represented by Formula (4a) described below.

When the X¹ group is an oxiranyl group, the compound represented by Formula (4) is represented by Formula (4a) and has one oxirane structure at each of both terminals. The L¹ group is the same as the definition of the L¹ group in Formula (4). A preferred crosslinking agent is a glycidyl ether of α,ω-alkanediol, and examples thereof include ethylene glycol diglycidyl ether, propanediol diglycidyl ether, 1,4-butanediol diglycidyl ether, and 1,5-pentanediol diglycidyl ether.

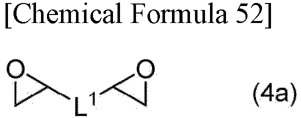

By reacting with the compound represented by Formula (3), a compound represented by Formula (1a-1), (1a-2), or (1a-3) is obtained. In Formula (1a-1), (1a-2), or (1a-3), CD, L³, m, and R^{A} correspond to the chemical structure of the compound represented by Formula (3) to be used, and L¹ corresponds to the chemical structure of the compound represented by Formula (4a) to be used.

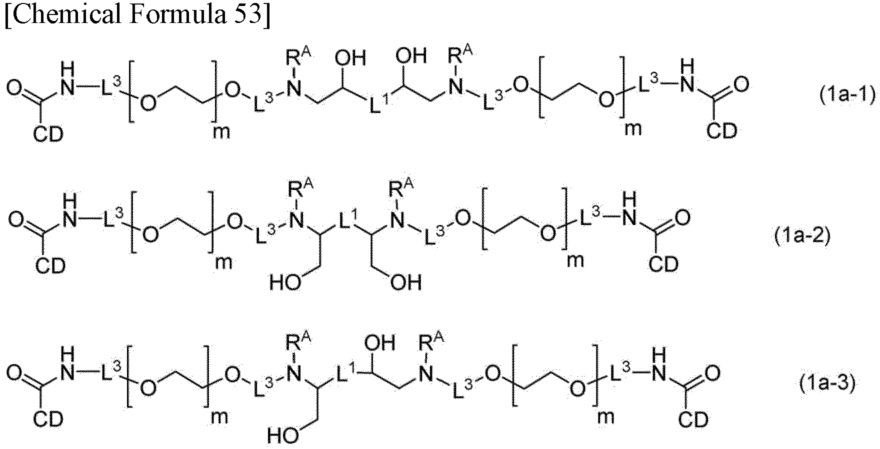

When the X¹ group is a formyl group, the compound represented by Formula (4) is represented by Formula (4b) and has one formyl group at each of both terminals. The L¹ group is the same as the definition of the L¹ group in Formula (4). A preferred crosslinking agent is glutaraldehyde.

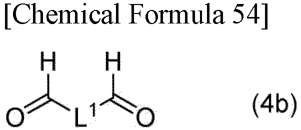

By reacting with the compound represented by Formula (3), a compound represented by Formula (1b) is obtained. When the compound represented by Formula (4b) is used as a crosslinking agent, the compound represented by Formula (3) needs to be a primary amine (in Formula (3), the R^{A} group is a hydrogen atom). In Formula (1b), CD, L³, and m correspond to the chemical structure of the compound represented by Formula (3) to be used, and L¹ corresponds to the chemical structure of the compound represented by Formula (4b) to be used.

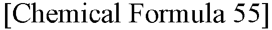

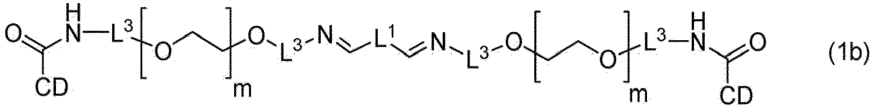

When the X¹ group is an isocyanate group, the compound represented by Formula (4) is represented by Formula (4c) and has one isocyanate group at each of both terminals. The L¹ group is the same as the definition of the L¹ group in Formula (4). A preferred crosslinking agent is 1,6-hexane diisocyanate.

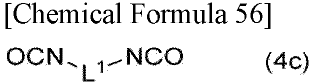

By reacting with the compound represented by Formula (3), a compound represented by Formula (1c) is obtained. In Formula (1c), CD, L³, m, and R^{A} correspond to the chemical structure of the compound represented by Formula (3) to be used, and L¹ corresponds to the chemical structure of the compound represented by Formula (4c) to be used.

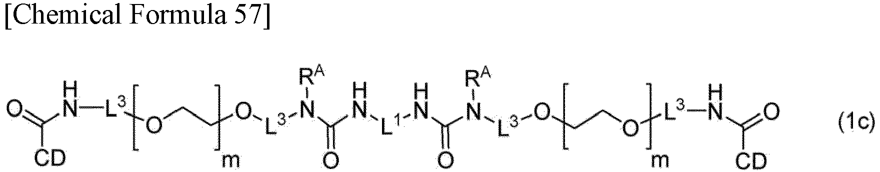

When the X¹ group is a succinimidyloxycarbonyl group or a 3-sulfosuccinimidyloxycarbonyl group, the compound represented by Formula (4) is represented by Formula (4d) and has one succinimidyloxycarbonyl group or 3-sulfosuccinimidyloxycarbonyl group at each of both terminals. The L¹ group is the same as the definition of the L¹ group in Formula (4). The L⁴ group is a hydrogen atom or a sulfo group. A preferred crosslinking agent is disuccinimidyl suberate, bis(3-sulfosuccinimidyl) suberate, 3,3'-dithiobis(3-sulfosuccinimidyl propionate), bis(succinimidyl) penta(ethylene glycol), bis(succinimidyl) nona(polyethylene glycol), or polyethylene glycol-modified bis(sulfosuccinimidyl) suberate.

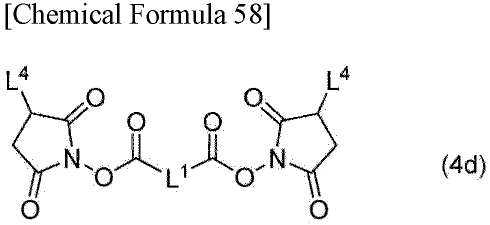

By reacting with the compound represented by Formula (3), a compound represented by Formula (1d) is obtained. In Formula (1d), CD, L³, m, and R^{A} correspond to the chemical structure of the compound represented by Formula (3) to be used, and L¹ corresponds to the chemical structure of the compound represented by Formula (4d) to be used.

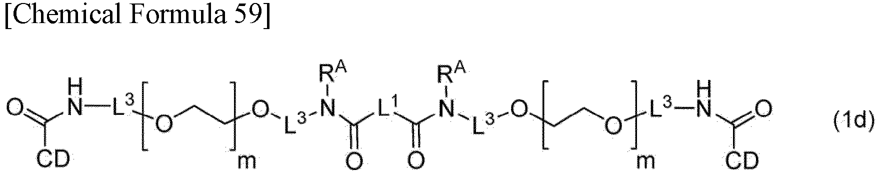

When the X¹ group is a carboxyimidate group, the compound represented by Formula (4) is represented by Formula (4e) and has one carboxyimidate group at each of both terminals. The L¹ group is the same as the definition of the L¹ group in Formula (4). In the case of using the crosslinking agent represented by Formula (4e), the reactivity of the crosslinking reaction may be higher when the pH of the reaction system is closer to 8. A preferred crosslinking agent is dimethylpimelimide dihydrochloride or dimethylsberimide dihydrochloride.

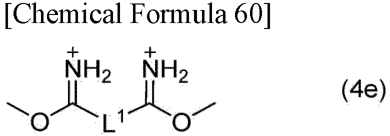

By reacting with the compound represented by Formula (3), a compound represented by Formula (1e) is obtained. In Formula (1e), CD, L³, m, and R^{A} correspond to the chemical structure of the compound represented by Formula (3) to be used, and L¹ corresponds to the chemical structure of the compound represented by Formula (4e) to be used.

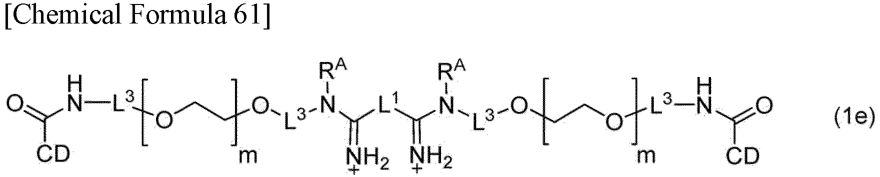

When the X¹ group is a carbonate group, the compound represented by Formula (4) is represented by Formula (4f) and has one carbonate group at each of both terminals. The L¹ group is the same as the definition of the L¹ group in Formula (4). The R^{X} group is a succinimidyloxycarbonyl group or a 3-sulfosuccinimidyloxycarbonyl group. In the case of using the crosslinking agent represented by Formula (4f), the reactivity of the crosslinking reaction may be higher when the pH of the reaction system is closer to 8. A preferred crosslinking agent is disuccinimidyl carbonate.

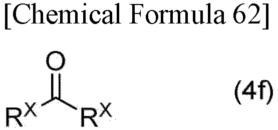

By reacting with the compound represented by Formula (3), a compound represented by Formula (1f) is obtained. In Formula (1f), CD, L³, m, and R^{A} correspond to the chemical structure of the compound represented by Formula (3) to be used.

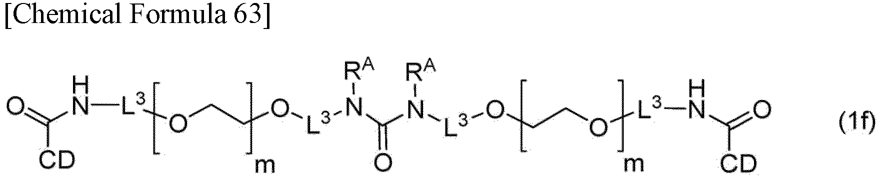

The amount of the crosslinking agent used may be 0.01 to 5 mol, and is preferably 0.1 to 3 mol, 0.1 to 2 mol, or 0.2 to 2 mol, with respect to 1 mol of the terminal amino group contained in the compound represented by Formula (3X) described below. When the amount of the crosslinking agent used is 0.1 to 3 mol with respect to 1 mol of the terminal amino group contained in the compound represented by Formula (3X), the content of the terminal amino group (unreacted amino group) contained in the polyrotaxane B represented by Formula (1X) tends to increase, and cell adhesiveness or tissue adhesiveness of the polyrotaxane B can be further enhanced. When the amount of the crosslinking agent used is 0.1 to 3 mol with respect to 1 mol of the terminal amino group contained in the compound represented by Formula (3X), a non-brittle gel excellent in handleability is obtained.

In this step, all the terminal amino groups contained in the polyrotaxane A represented by Formula (3X) may not react (crosslinking reaction) with the crosslinking agent. When the terminal amino group did not react with the crosslinking agent, the polyrotaxane B contains both the compound represented by Formula (1) and the compound represented by Formula (3), and has the chemical structure represented by Formula (1Xa).

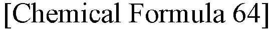

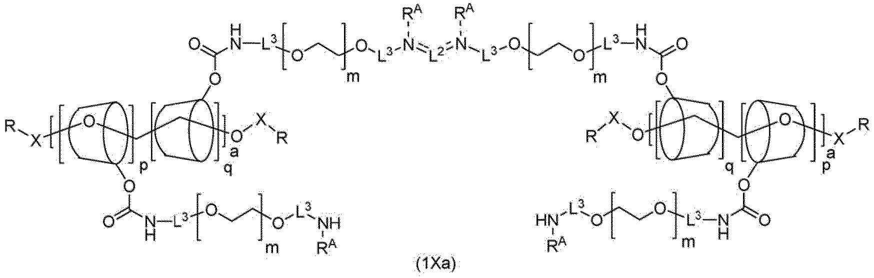

R, X, L², L³, R^{A}, m, and a in Formula (1Xa) are each as defined in Formula (1X), p is any integer of 0 to n, and q is any integer of 1 to n.

The method for producing a polyrotaxane according to the present embodiment may further comprises a step of mixing a polyrotaxane represented by Formula (5) (polyrotaxane in which CD is not modified) and polyoxyethylenediamine in the presence of a carbonylating agent to obtain a compound represented by Formula (3X).

In this step, by reacting the polyrotaxane represented by Formula (5) with a diamine represented by Formula (6) in the presence of a carbonylating agent, an oxygen atom constituting a hydroxyl group of CD and a nitrogen atom of polyoxyethylenediamine are bonded via a carbonyl group to form a urethane bond. Thereafter, the R^{A} group may be optionally introduced by alkylation of the terminal primary amino group. The following formula is a chemical formula schematically representing this step. In the formula, R, X, L³, R^{A}, m, n, and a are as defined in Formula (1) or Formula (2).

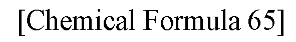

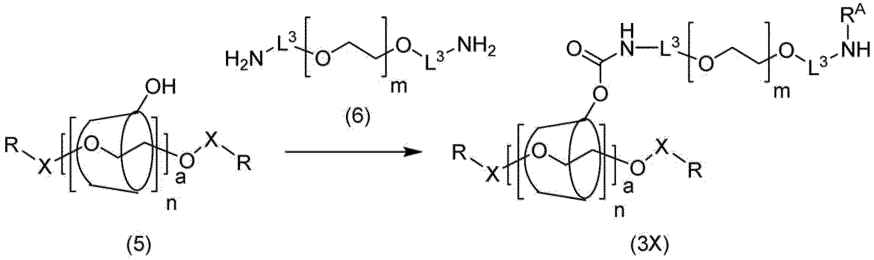

The carbonylating agent may be a compound in which two leaving groups are bonded to a carbon atom of a carbonyl group, and examples thereof include 1,1-carbonyldiimidazole (CDI), phosgene, and triphosgene. A preferred carbonylating agent is CDI. The amount of the carbonylating agent used may be 1 to 20 mol, and is preferably 2 to 15 mol, with respect to 1 mol of the cyclodextrin contained in the polyrotaxane represented by Formula (5).

The diamine represented by Formula (6) is a polyoxyalkylene having an amino group at both terminals, and the number m of oxyethylene units is as defined in Formula (1). L³ is an ethylene group or a propylene group. Examples of the diamine represented by Formula (6) include 3,3'-diaminopropyl ether and polyethylene glycol diamine. The amount of the diamine represented by Formula (6) used may be 10 to 400 mol, and is preferably 20 to 200 mol, with respect to 1 mol of the cyclodextrin contained in the polyrotaxane represented by Formula (5).

This step is preferably performed in an organic solvent. The organic solvent may be any solvent as long as it does not suppress or inhibit the present reaction, and examples thereof include tetrahydrofuran (THF), tert-butyl methyl ether (TBME), cyclopentyl methyl ether (CPME), and dimethyl sulfoxide (DMSO). A preferred organic solvent is DMSO. The amount of the organic solvent used may be 10 to 200 mL, and is preferably 20 to 100 mL, with respect to 1 g of the polyrotaxane.

This step can be performed within a range of 0 to 70°C, and may be performed at room temperature (for example, 0 to 30°C). The reaction solution may be appropriately heated in order to accelerate the reaction.

The number of introduced side chains (side chains derived from a diamine represented by Formula (6)) can be calculated by ¹H-NMR analysis.

As the reaction of introducing the R^{A} group into the terminal amino group, an N-alkylation reaction well known in the field of organic chemistry can be applied. Examples of the alkylating agent include C₁₋₆ alkanes having a leaving group, such as C₁₋₆ alkyl halide, C₁₋₆ alkanol mesylate, and C₁₋₆ alkanol tosylate. In the presence of formaldehyde or a C₁₋₅ alkyl aldehyde, a borohydride reagent such as sodium cyanoborohydride may be added and subjected to a reductive N-alkylation reaction.

### [Fourth Embodiment]

A fourth embodiment of the present invention is a polyrotaxane (polyrotaxane A) having a chemical structure in which an axial molecule represented by Formula (2) is threaded into each cyclodextrin ring in one or a plurality of compounds represented by Formula (3). The polyrotaxane A has the chemical structure represented by Formula (3X).

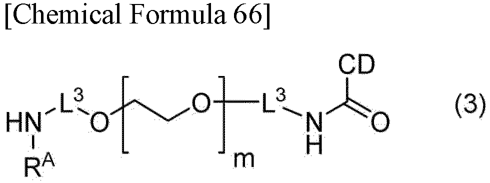

In Formula (3), CD is a cyclodextrin ring, L³ is an ethylene group or a propylene group, R^{A} is a hydrogen atom or a C₁₋₆ alkyl group, and m is an integer of 0 to 35.

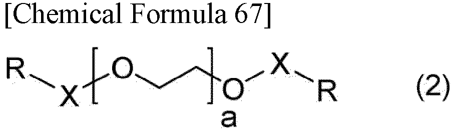

In Formula (2), R is a hydrocarbon group larger than an inner diameter of the cyclodextrin ring, X is a divalent organic group, and a is an integer of 100 to 1000.

The polyrotaxane A according to the present embodiment is excellent in water solubility, and is suitable for use as a part of a kit according to the fourth embodiment.

### [Fifth Embodiment]

A fifth embodiment of the present invention is a kit comprising a polyrotaxane (polyrotaxane A) having a chemical structure in which an axial molecule represented by Formula (2) is threaded into each cyclodextrin ring in one or a plurality of compounds represented by Formula (3) and a crosslinking agent represented by Formula (4).

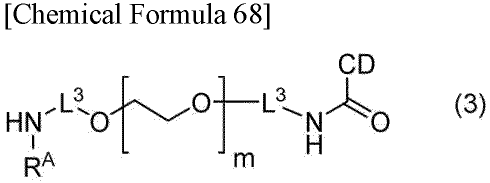

In Formula (3), CD is a cyclodextrin ring, L³ is an ethylene group or a propylene group, R^{A} is a hydrogen atom or a C₁₋₆ alkyl group, and m is an integer of 0 to 35.

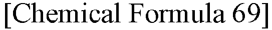

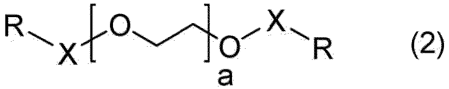

In Formula (2), R is a hydrocarbon group larger than an inner diameter of the cyclodextrin ring, X is a divalent organic group, and a is an integer of 100 to 1000.

As described in the third embodiment, the polyrotaxane B is produced by mixing the polyrotaxane A and the crosslinking agent represented by Formula (4). For a method for using the kit according to the present embodiment, the description of the third embodiment can be referred to.

The polyrotaxane A and the crosslinking agent represented by Formula (4) contained in the kit may be in the state of a composition (for example, a solution or a dispersion) as long as they are in the state of being separated so that they can be mixed at the time of use. When these two components are in the state of a composition, they are easily mixed at the time of use, and the practicality is further enhanced. As a preferred aspect, there is provided a kit comprising a composition comprising a polyrotaxane A and a composition comprising a crosslinking agent represented by Formula (4), and these two compositions may be contained in separate containers, or may be contained in separate chambers in a container including a plurality of chambers. When these two compositions are used, they may be mixed manually or may be applied to a target biological tissue using a syringe equipped with a static mixer. Since the crosslinking reaction proceeds rapidly, the compositions may be prepared at need. Since the crosslinking reaction proceeds even at room temperature to body temperature (around about 37°C), the composition comprising a polyrotaxane A and the composition comprising a crosslinking agent represented by Formula (4) each may be applied to a biological tissue to be used and may be mixed.

### [Examples]

Hereinafter, the present invention will be described in more detail with reference to Examples and Comparative Examples. Note that, abbreviations used in Examples should be understood as commonly understood by those skilled in the art, for example, as follows.
αCD: α-cyclodextrin
BOP: benzotriazolyl-N-hydroxytrisdimethylaminophosphonium hexafluorophosphate
CDI: 1,1-carbonyldiimidazole
DMF: dimethylformamide
DMSO: dimethyl sulfoxide
DMT-MM: 4-(4,6-dimethoxy-1,3,5-triazine-2-yl)-4-methylmorpholinium chloride
EDIPA: N,N-diisopropylethylamine
EGDE: ethylene glycol diglycidyl ether
BDDE: 1,4-butanediol diglycidyl ether
HOBt: 1-hydroxybenzotriazole
MilliQ water: ultrapure water purified by an ultrapure apparatus Milli-Q
TEMPO: 2,2,6,6-tetramethylpiperidine-N-oxide
THF: tetrahydrofuran

¹H-NMR is corrected using tetramethylsilane as an internal standard and is expressed using chemical shifts (unit: ppm).

### 1. Production of Polyrotaxane

### Production Example 1

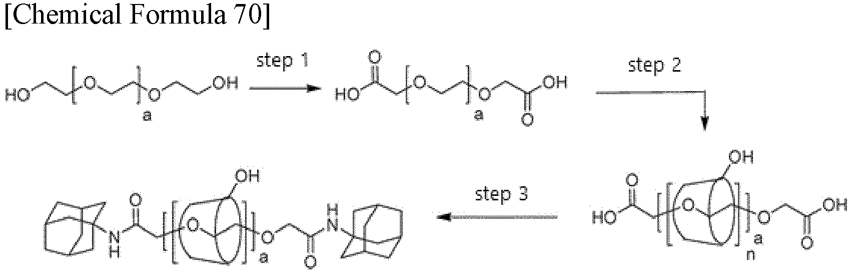

### [Step 1]

Polyethylene glycol (20.0 g, 1.0 mmol) having a number average molecular weight of 20000 was dissolved in water (340 mL), TEMPO (330 mg, 2.11 mmol), sodium bromide (330 mg, 3.21 mmol), and sodium hypochlorite (content: 4.0%, 40 mL) were added thereto, it was confirmed that the reaction solution became alkaline (pH = 9), and stirring was performed at 23°C. After 20 minutes, ethanol (40 mL) and 2 M hydrochloric acid (6 mL) were added dropwise, and it was confirmed that the reaction solution became acidic (pH = 2). The reaction solution was extracted with methylene chloride, the solvent of the organic layer thus obtained was distilled off, and then ethanol (200 mL) at 50°C was added thereto for dissolution. The obtained solution was cooled to 4°C to precipitate crystals, and then the solution containing the crystals was filtered with cooled ethanol to obtain a residue thereof. The residue was dissolved again in ethanol at 50°C, cooled to precipitate crystals, and then filtered with cooled ethanol. The obtained residue was dried under reduced pressure to obtain dicarboxylic acid (14.5 g, yield: 73%).

### [Step 2]

The dicarboxylic acid (10.0 g, 0.500 mmol) obtained in Step 1 was dissolved in water (300 mL), a saturated aqueous solution (45 mL) of αCD (50.0 g, 51.4 mmol) was added thereto, and stirring was performed at 23°C. After 24 hours, the reaction solution was centrifuged to collect a precipitate, and freeze-dried for 3 days to obtain a pseudo-polyrotaxane as a crude product.

### [Step 3]

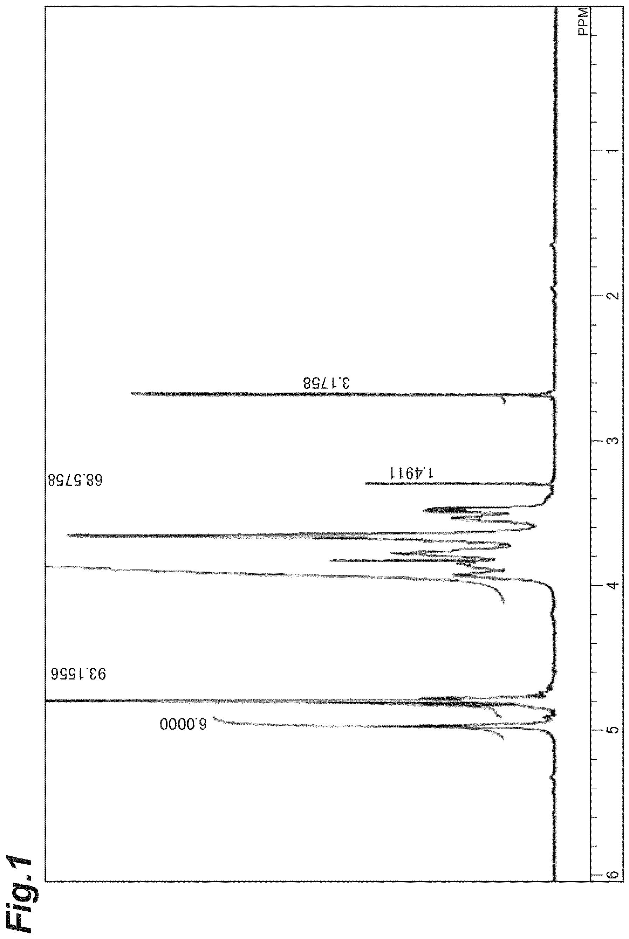

1-Adamantanamine (13.8 g, 91.5 mmol) was dissolved in super dehydrated DMF (340 ml), the pseudo-polyrotaxane (51.4 g) obtained in Step 2 and DMT-MM (22.6 g, 81.5 mmmol) were added to the reaction solution, and stirring was performed at 23°C. After 24 hours, the crude product was washed with water and methanol, reprecipitated with hydrous DMSO, centrifuged and freeze-dried for 4 days to obtain a polyrotaxane (32.4 g, yield in Step 2: 54%) of Production Example 1 as a powder. The ¹H-NMR spectrum (frequency: 500 MHz, solvent: D₂O + NaOD) of the obtained polyrotaxane is shown in FIG. 1.

It was confirmed by GPC analysis that non-threaded αCD was removed.
Measurement conditions
Apparatus: Prominence-i LC-2030 Plus (manufactured by SHIMADZU CORPORATION)
Detector: RID-20A refractive index detector (manufactured by SHIMADZU CORPORATION)
Column: TSK gel (registered trademark) α-4000 and α-2500 (length: 300 mm, inner diameter: 7.8 mm) (manufactured by Tosoh Corporation)
Column temperature: 60°C
Flow rate: 0.35 mL/min

### Production Examples 2A to 2C

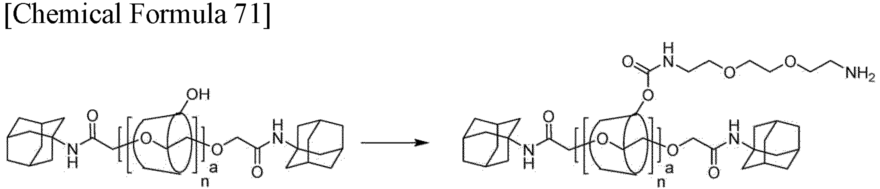

### [Step 1]

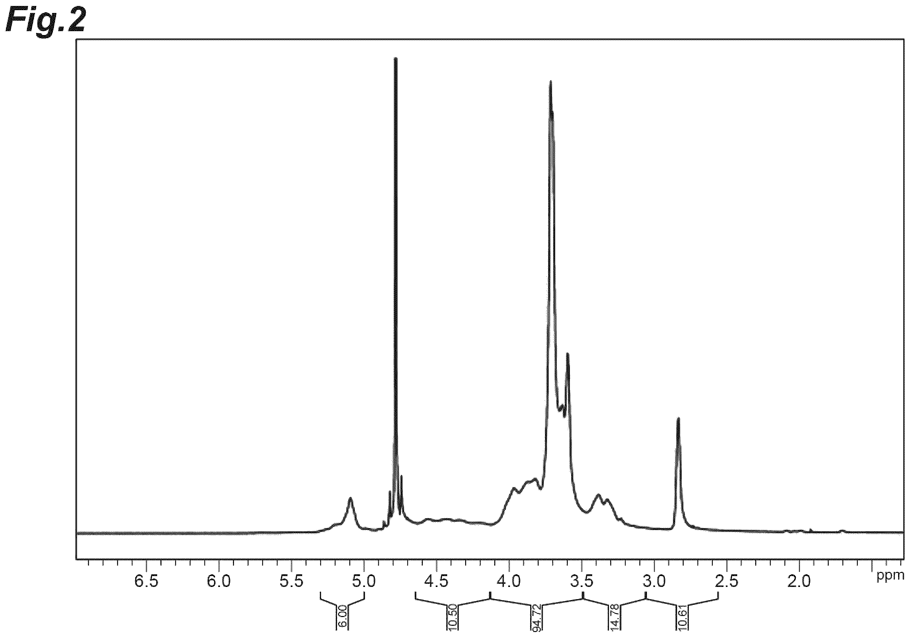

Polyrotaxane (8.00 g, 0.108 mmol) of Production Example 1 was dissolved in anhydrous DMSO (250 mL), CDI (7.79 g, 48.0 mmol) was added thereto, and the reaction solution was stirred at 23°C. After 1 day, the reaction solution was added dropwise to 1,2-bis(2-aminoethoxyethane) (71 mL, 0.48 mol), and stirring was further performed at 23°C for 1 day. Next, the reaction solution was purified by dialysis for 5 days. The product was freeze-dried for 2 days to obtain polyrotaxane (7.19 g, yield: 53%) of Production Example 2B as a powder. The number of introduced side chains was determined by ¹H-NMR analysis (frequency: 500 MHz, solvent: D₂O + NaOD). ¹H-NMR of the polyrotaxane of Production Example 2B is shown in FIG. 2.

### Production Example 3

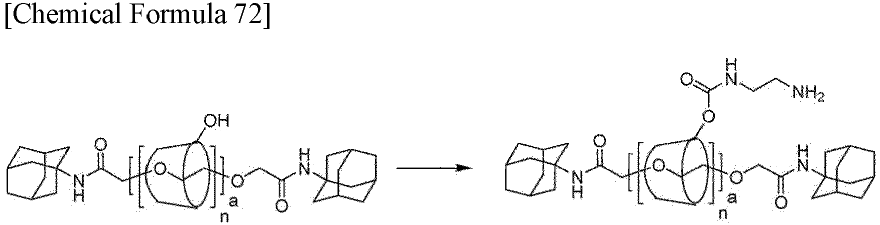

### [Step 1]

Polyethylene glycol (20.0 g, 1.0 mmol) having a number average molecular weight of 20000 was dissolved in water (340 mL), TEMPO (330 mg, 2.11 mmol), sodium bromide (330 mg, 3.21 mmol), and sodium hypochlorite (content: 4.0%, 40 mL) were added thereto, it was confirmed that the reaction solution became alkaline (pH = 9), and stirring was performed at 23°C. After 20 minutes, ethanol (40 mL) and 2 M hydrochloric acid (6 mL) were added dropwise, and it was confirmed that the reaction solution became acidic (pH = 2). The reaction solution was extracted with methylene chloride, the solvent of the organic layer thus obtained was distilled off, and then ethanol (200 mL) at 50°C was added thereto for dissolution. The obtained solution was cooled to 4°C to precipitate crystals, and then the solution containing the crystals was filtered with cooled ethanol to obtain a residue thereof. The residue was dissolved again in ethanol at 50°C, cooled to precipitate crystals, and then filtered with cooled ethanol. The obtained residue was dried under reduced pressure to obtain dicarboxylic acid (14.5 g, yield: 73%).

### [Step 2]

The dicarboxylic acid (5.0 g, 0.25 mmol) obtained in Step 1 was dissolved in water (20 mL), a saturated aqueous solution (150 mL) of αCD (25.0 g, 25.7 mmol) was added thereto, and stirring was performed at 23°C. After 24 hours, the reaction solution was centrifuged to collect a precipitate, and freeze-dried for 4 days to obtain a pseudo-polyrotaxane as a crude product.

### [Step 3]

1-Adamantanamine (6.92 g, 45.8 mmol) was dissolved in super dehydrated DMF (120 ml), the pseudo-polyrotaxane (22.0 g) obtained in Step 2 and DMT-MM (11.3 g, 40.8 mmmol) were added to the reaction solution, and stirring was performed at 23°C. After 24 hours, the crude product was washed with water and methanol, reprecipitated with hydrous DMSO, centrifuged and freeze-dried for 4 days to obtain a polyrotaxane (15.6 g, yield in Step 2: 52%) as a powder. The number of threaded αCDs was determined by ¹H-NMR (frequency: 500 MHz, solvent: D₂O + NaOD).

### [Step 4]

Polyrotaxane (4.00 g, 41.6 µmol) and CDI (5.28 g, 32.6 mmol) were dissolved in anhydrous DMSO (130 mL), and the reaction solution was stirred at 23°C. After 1 day, the reaction solution was added dropwise to ethylenediamine (44 mL, 0.652 mol), and stirring was further performed at 23°C for 1 day. Next, the reaction solution was purified by dialysis for 5 days. The product was freeze-dried for 11 days to obtain polyrotaxane (2.78 g, yield: 51%) of Production Example 3 as a powder. The number of amino groups was determined by ¹H-NMR (frequency: 500 MHz, solvent: D₂O + NaOD).

Data of the polyrotaxanes of Production Examples 1, 2A to 2C, and 3 are shown in Table 1. The number of introduced side chains per CD was calculated on the basis of ¹H-NMR analysis.

**[Table 1]**

| | PEG molecular weight | Number of threaded CDs | Number of introduced side chains per CD | Number of introduced side chains | Molecular weight of polyrotaxane |
|---|---|---|---|---|---|
| Production Example 1 | 20000 | 55.5 | - | - | 74100 |
| Production Example 2A | 20000 | 55.5 | 6.0 | 333 | 132000 |
| Production Example 2B | 20000 | 55.5 | 5.3 | 294 | 126000 |
| Production Example 2C | 20000 | 55.5 | 4.0 | 222 | 113000 |
| Production Example 3 | 20000 | 78.4 | 5.0 | 392 | 130000 |

### Production Example 4

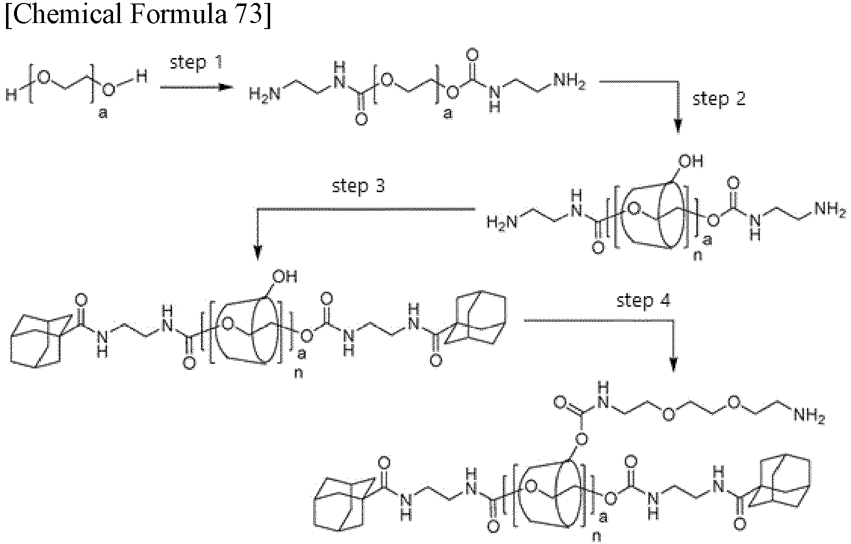

### [Step 1]

Polyethylene glycol (50.0 g, 2.5 mmol) having a number average molecular weight of 20000 was dissolved in THF (200 mL), CDI (1.0 g, 6.2 mmol) was added thereto, and the reaction solution was stirred at 50°C. After 18 hours, the reaction solution was added dropwise to ethylenediamine (3.0 mL, 44 mmol), and stirring was further performed at 50°C for 2 hours. Subsequently, ethanol (200 mL) was added, and the mixture was left to stand still at -20°C for 2 hours. The mixture was washed with cooled ethanol (200 mL), filtered, and freeze-dried to obtain diamine.

### [Step 2]

The obtained diamine (3.0 g, 0.15 mmol) was dissolved in water (100 mL), αCD (12.0 g, 12.3 mmol) was added thereto, and stirring was performed at 4°C. The reaction solution was centrifuged to collect a precipitate, and freeze-dried to obtain a pseudo-polyrotaxane as a crude product.

### [Step 3]

1-Adamantane carboxylic acid (2.45 g, 13.6 mmol), BOP (5.25 g, 11.9 mmol), HOBt (1.75 g, 12.6 mmol), and EDIPA (2.275 mL, 17.6 mmol) were dissolved in THF (100 mL), the pseudo-polyrotaxane (14.0 g) obtained in Step 2 was added thereto, and the reaction solution was stirred at 4°C. After 48 hours, the crude product was washed with DMF and methanol, reprecipitated with hydrous DMSO, centrifuged and freeze-dried to obtain a polyrotaxane as a powder. The number of threaded αCDs was determined by ¹H-NMR (frequency: 500 MHz, solvent: DMSO-d₆).

### [Step 4]

Polyrotaxane (8.41 g, 0.108 mmol) was dissolved in anhydrous DMSO (250 mL), CDI (8.29 g, 51.1 mmol) was added thereto, and the reaction solution was stirred at 23°C. After 1 day, the reaction solution was added dropwise to 1,2-bis(2-aminoethoxyethane) (76 mL, 0.51 mol), and stirring was further performed at 23°C for 1 day. Next, the reaction solution was purified by dialysis for 5 days. The product was freeze-dried to obtain a polyrotaxane of Production Example 4 as a powder.

Data of the polyrotaxane of Production Example 4 are shown in Table 2. The number of introduced side chains per CD was calculated on the basis of ¹H-NMR analysis.

**[Table 2]**

| | PEG molecular weight | Number of threaded CDs | Number of introduced side chains per CD | Number of introduced side chains | Molecular weight of polyrotaxane |
|---|---|---|---|---|---|
| Production Example 4 | 20000 | 59.3 | 3.9 | 231 | 118000 |

### 2. Evaluation of Water Solubility of Polyrotaxane

### [Method]

As shown in Table 3, a predetermined amount of a polyrotaxane powder of Production Example 4 was weighed and put in a glass bottle, then MilliQ water was added so as to have various concentrations (80, 160, or 320 mg/mL), and stirring was performed at 23°C for 1 hour to prepare a polyrotaxane aqueous solution. A polyrotaxane aqueous solution (concentration: 40 or 80 mg/mL) of Production Example 3 was prepared in the same manner. The light transmittance (%T) of each of polyrotaxane aqueous solutions 1 to 6 was measured using a spectrophotometer (product name: U-2910 manufactured by Hitachi High-Tech Science Corporation).

**[Table 3]**

| No. | Polyrotaxane | | Temperature |
|---|---|---|---|
| | Type | Concentration | |
| 1 | Production Example 3 | 40 mg/mL | 50°C |
| 2 | | 80 mg/mL | 50°C |
| 3 | | 40 mg/mL | Room temperature |
| 4 | | 80 mg/mL | Room temperature |
| 5 | Production Example 4 | 80 mg/mL | Room temperature |
| 6 | | 160 mg/mL | Room temperature |
| 7 | | 320 mg/mL | Room temperature |

### [Results]

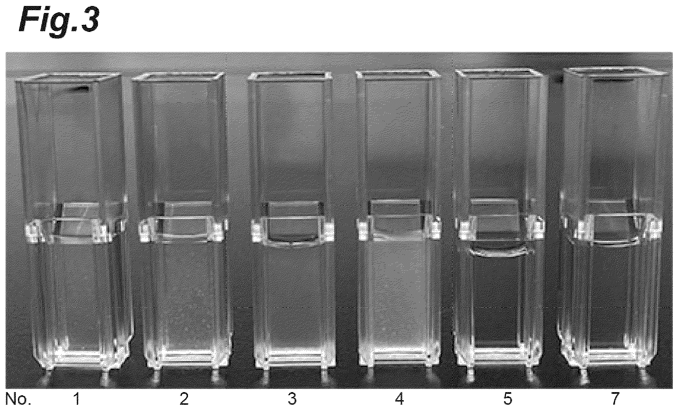

Each polyrotaxane aqueous solution was placed in an optical cell, and an appearance photograph thereof was taken. The photographs are shown in FIG. 3. Since the polyrotaxane of Production Example 1 (unmodified) was insoluble in water, the transmittance was not measured.

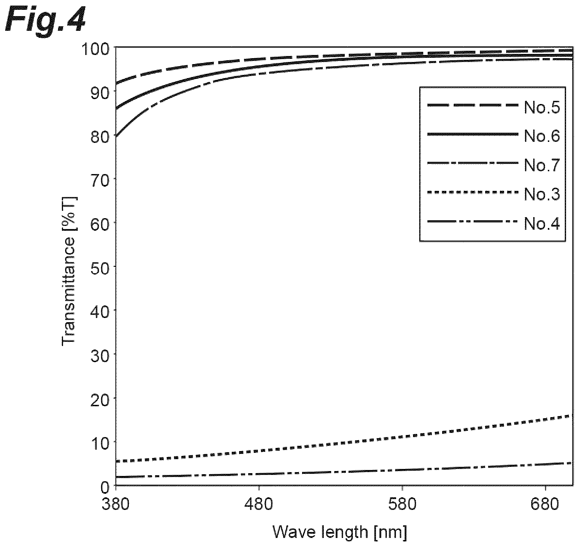

The results of the light transmittance at a wavelength of 600 nm are shown in Table 4. The polyrotaxane aqueous solution of Production Example 4 was transparent even at a concentration of 320 mg/mL, and exhibited excellent water solubility. On the other hand, the polyrotaxane aqueous solution of Production Example 3 was cloudy even at a concentration of 40 mg/mL, and had not very high water solubility. The polyrotaxane aqueous solution of Production Example 3 did not become transparent even when being heated to 50°C. The total transmittance spectra in the visible light region (wavelength: 380 to 780 nm) are shown in FIG. 4. The polyrotaxane of Production Example 4 exhibited a high light transmittance over the entire measured wavelength range.

**[Table 4]**

| No. | Polyrotaxane | | Transmittance [600 nm] |
|---|---|---|---|
| | Type | Concentration | |
| 3 | Production Example 3 | 40 mg/mL | 11.8 |
| 4 | | 80 mg/mL | 4 |
| 5 | Production Example 4 | 80 mg/mL | 98.2 |
| 6 | | 160 mg/mL | 96.9 |
| 7 | | 320 mg/mL | 96.3 |

### 3. Evaluation of Gelation Time of Polyrotaxane Derivative

### [Gelation]

A predetermined amount of a polyrotaxane powder of Production Example 4 was weighed and put in a glass bottle, then MilliQ water was added so as to have various concentrations (80 mg/mL, 160 mg/mL, or 320 mg/mL), and stirring was performed at 23°C for 1 hour to prepare a polyrotaxane aqueous solution. A crosslinking agent EGDE was added to the obtained polyrotaxane aqueous solution, stirring was performed for 20 seconds using a vortex mixer, and then stirring was performed using a stirrer (rotation speed: 320 rpm) under an environment of 37°C. To the number of cyclodextrins (CDs) contained in the used polyrotaxane, 1.4 molar equivalents or 3.5 molar equivalents of the crosslinking agent was added. The time from the addition of the crosslinking agent until the rotation of the stirrer bar stopped was measured and recorded as the gelation time. The gelation time of the polyrotaxane of Production Example 3 was also evaluated in the same manner.

### [Results]

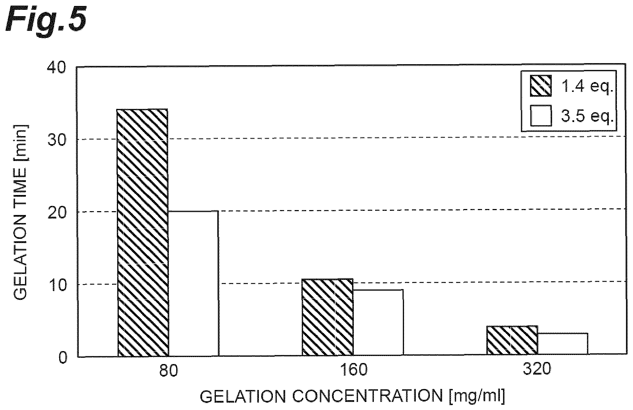

The results are shown in Table 5 and FIG. 5. The gelation time decreased with increasing the polyrotaxane concentration. In the case of the polyrotaxane of Production Example 4, it was found that when the polyrotaxane concentration was 320 mg/mL, gelation was possible within 5 minutes at 37°C. At the same concentration, when 3.5 equivalents of EGDE was added to the polyrotaxane, a slightly cloudy hydrogel was obtained. The present inventors consider that this is because EGDE used as the crosslinking agent is hardly dissolved in water. On the other hand, when the polyrotaxane of Production Example 3 was used, a hydrogel to be obtained was cloudy even at a concentration of 80 mg/mL.

**[Table 5]**

| | Polyrotaxane | | Equivalents [eq.] of EGDE with respect to CD | Gelation time |
|---|---|---|---|---|
| | Type | Concentration | | |
| Example 1 | Production Example 4 | 80 mg/mL | 1.4 | 34 |
| Example 2 | | | 3.5 | 20 |
| Example 3 | | 160 mg/mL | 1.4 | 10.5 |
| Example 4 | | | 3.5 | 9 |
| Example 5 | | 320 mg/mL | 1.4 | 4 |
| Example 6 | | | 3.5 | 3 |
| Comparative Example 2 | Production Example 3 | 80 mg/mL | 1.2 | 22 |

### (2) Evaluation of Cell Adhesiveness of Hydrogel

A polystyrene dish for cell culture was coated with a mixed solution of the polyrotaxane aqueous solution of Production Example 2A and the crosslinking agent to produce a hydrogel. Mouse fibroblasts were seeded on an uncoated polystyrene dish or hydrogel coating, and cell adhesiveness was evaluated by taking a picture of the cells once a day until 1 week later.

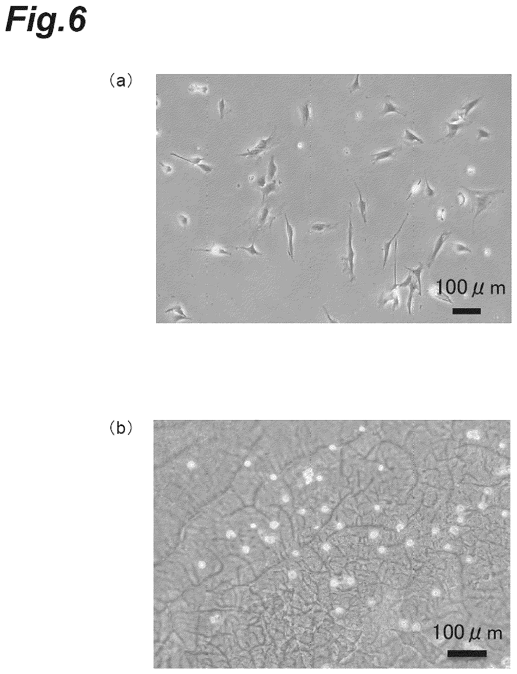

The results are shown in FIG. 6. FIG. 6 shows photographs of mouse fibroblasts seeded on a polystyrene dish. In a normal polystyrene dish (without coating, FIG. 6(a)), fibroblasts were observed to adhere and extend and to proliferate after one week. On the other hand, in a coated polystyrene dish (FIG. 6(b)), although fibroblast adhered, almost no extension was observed, and almost no extension cell was observed and no remarkable proliferation was observed even after a lapse of one week. From these results, it was found that the hydrogel coating can adhere fibroblasts in a living state, and hardly causes activation and proliferation thereof.

### 3. Evaluation of Tissue Adhesiveness of Hydrogel

### [Preparation of Hydrogel]

A crosslinking agent was added to a polyrotaxane aqueous solution of Production Example 2A having a concentration of 100 mg/mL so as to be 3.5 molar equivalents with respect to the number of αCDs contained in the polyrotaxane, and stirring was performed. The reaction solution was rapidly poured into a mold and left to stand still at 23°C to obtain a hydrogel. After 1 day, the hydrogel was removed from the mold and washed with MilliQ water.

### [Tissue Adhesiveness Test]

As tissue specimens, cecum, uterus, and small intestine of pigs (purchased from Tokyo Shibaura Organ Co., Ltd.) were used. The hydrogel was attached to each tissue specimen so that the hydrogel was sandwiched. Adhesion and appearance of the hydrogel were observed immediately after, 1 hour after, and 1 day after attaching the hydrogel. For comparison, SEPRAFILM (registered trademark, Sanofi K.K.) was used instead of the hydrogel.

### [Results]

The hydrogel adhered quickly after being attached, and adhered even after 1 hour and even after 1 day. SEPRAFILM adhered rapidly and strongly after being attached, but immediately changed from a sheet shape to a gel shape, and after 1 hour, the material became brittle, and the adhesiveness was reduced.

### 4. Evaluation of Transparency of Hydrogel

### [Method]

A predetermined amount of a polyrotaxane powder of Production Example 4 was weighed and put in a glass bottle, then MilliQ water was added so as to have various concentrations (80, 160, or 320 mg/mL), and stirring was performed at 23°C for 1 hour to prepare a polyrotaxane aqueous solution. After mixing the polyrotaxane aqueous solution of Production Example 4 and the crosslinking agent (EGDE or BDDE) in a 2.0 mL tube, 0.8 mL of the reaction solution was transferred to an optical cell and left to stand still at 23°C for 1 day (gelation). MilliQ water was added to the reaction solution to wash the gel, and MilliQ water was added and left to stand still at 23°C for another 1 day.

In the same manner, a predetermined amount of a polyrotaxane powder of Production Example 3 was weighed and put in a glass bottle, then MilliQ water was added so as to have various concentrations (80 mg/mL), stirring was performed at 23°C for 1 hour, and then the solution was immersed in a water bath at 50°C for 20 minutes to prepare a polyrotaxane aqueous solution. After mixing the polyrotaxane aqueous solution of Production Example 3 and a crosslinking agent EGDE in a 2.0 mL tube, 0.8 mL of the reaction solution was transferred to an optical cell and left to stand still at 23°C for 1 day (gelation). MilliQ water was added to the reaction solution to wash the gel, and MilliQ water was added and left to stand still at 23°C for another 1 day.

An appearance photograph of each hydrogel was taken. The light transmittance (%T) of the obtained hydrogel was measured using a spectrophotometer (product name: U-2910 manufactured by Hitachi High-Tech Science Corporation). The light transmittance at a wavelength of 600 nm and the total transmittance in the visible light region (380 to 780 nm) of each hydrogel were compared.

### [Results]

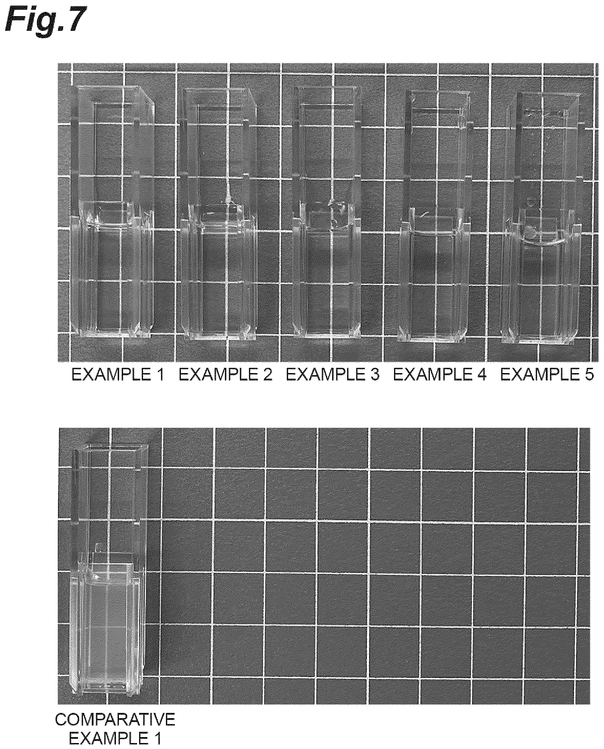

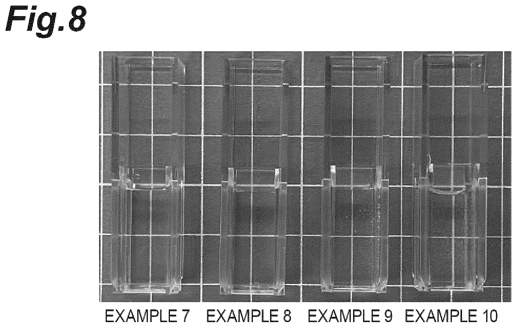

Appearance photographs of each of hydrogels are shown in FIGS. 7 and 8. In the case of using the polyrotaxane of Production Example 4, a transparent hydrogel was obtained. In Examples 9 and 10, the viscosity of the hydrogel was high, and air bubbles were mixed, but the hydrogel itself was transparent. On the other hand, in the case of using the polyrotaxane of Production Example 3, the hydrogel was cloudy even at a low concentration.

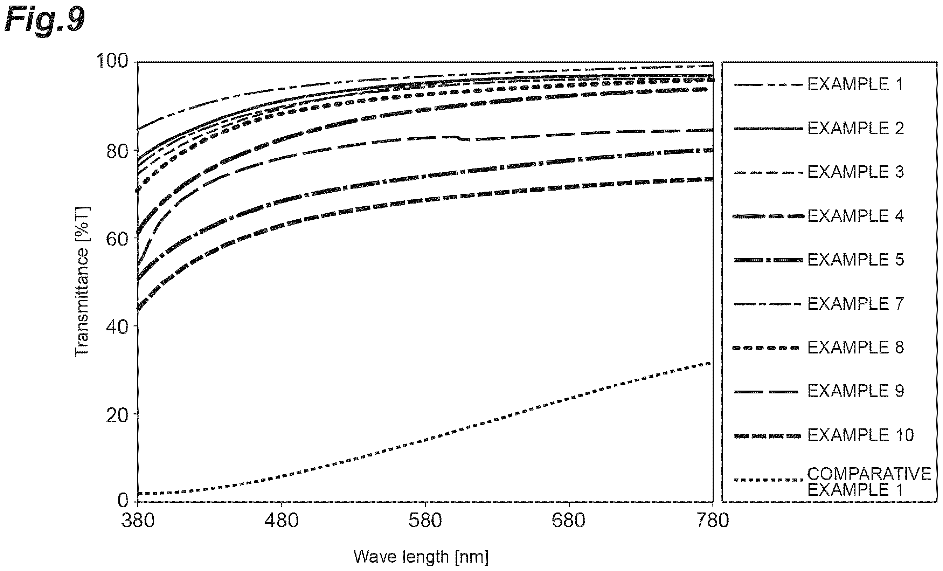

The results of the light transmittance of each hydrogel at a wavelength of 600 nm or in the visible light region are shown in Tables 6 and 7. The total transmittance spectra in the visible light region (wavelength: 380 to 780 nm) are shown in FIG. 9. The hydrogel derived from the polyrotaxane of Production Example 4 showed a high transmittance even at a high concentration (320 mg/ml), but the hydrogel derived from the polyrotaxane of Production Example 3 was cloudy and also had a low transmittance even at 80 mg/ml.

**[Table 6]**

| | Polyrotaxane | | Equivalents [eq.] of EGDE with respect to CD | Transmittance [600 nm] | Transmittance [380 to 780 nm] |
|---|---|---|---|---|---|
| | Type | Concentration | | | |
| Example 1 | Production Example 4 | 80 mg/mL | 1.4 | 96.7 | 95 |
| Example 2 | | | 3.5 | 94.5 | 92 |
| Example 3 | | 160 mg/mL | 1.4 | 93.3 | 91 |
| Example 4 | | | 3.5 | 89.4 | 86 |
| Example 5 | | 320 mg/mL | 1.4 | 74.8 | 72 |
| Comparative Example 1 | Production Example 3 | 80 mg/mL | 1.4 | 15.8 | 15 |

**[Table 7]**

| | Polyrotaxane | | Equivalents [eq.] of EGDE with respect to CD | Transmittance [600 nm] | Transmittance [380 to 780 nm] |
|---|---|---|---|---|---|
| | Type | Concentration | | | |
| Example 7 | Production Example 4 | 80 mg/mL | 1.4 | 93.6 | 91 |
| Example 8 | | 160 mg/mL | 1.4 | 93.5 | 91 |
| Example 9 | | 320 mg/mL | 1.4 | 82.6 | 79 |
| Example 10 | | | 3.5 | 69.2 | 66 |

### 5. Evaluation of Adhesion Preventing Property of Hydrogel

### [Production of Hydrogel]

A crosslinking agent EGDE was added to a polyrotaxane aqueous solution (concentration: 160 mg/mL) of Production Example 2B so as to be 1.4 molar equivalents with respect to the number of αCDs contained in the polyrotaxane, and stirring was performed. The reaction solution was rapidly poured into a mold made of silicone rubber (volume: about 0.2 mL) to fill the mold, and then covered with a film so that air bubbles did not enter, and the mold was left to stand still at 23°C. After 1 day, the hydrogel was removed from the mold and washed with MilliQ water to obtain a hydrogel with a size of 20 mm × 20 mm and a thickness of 0.5 mm.

### [Preparation of Mouse]

The adhesion preventing function was evaluated using a cecal abrasion adhesion model of a mouse in accordance with the contents approved by Institutional Animal Care and Use Committee of Tokyo Medical and Dental University. The abdomen of the mouse was open, the cecum was abraded with a commercially available toothbrush, the hydrogel was then placed, and the incision was sutured (n = 4). For comparison, a sham group (the abdomen of the mouse was open, and then the incision was sutured without abrasion), a non-treated group (the abdomen of the mouse was open, the cecum was then abraded with a toothbrush, and the incision was sutured), and a SEPRAFILM group (the abdomen of the mouse was open, the cecum was then abraded with a toothbrush, SEPRAFILM was placed, and the incision was sutured) were prepared (n = 4 for each group). The adhesion preventing function was evaluated by comparing the degree of adhesion one week after suturing. As with the hydrogel, SEPRAFILM was cut into a size of 20 mm × 20 mm and used.

### [Evaluation]

For each treatment group, with reference to the description of PLoS One. 2019; 14(1): e0211391., the degree (Severity Score) and the area (Area Score) of adhesion were evaluated in five stages, and the average value thereof was recorded. That is, the evaluation criteria are as follows.

### <Evaluation Criteria of Degree of Adhesion>

0: No adhesion
1: Mild adhesion
2: Moderate localized adhesion
3: Moderate and extensive adhesion
4: Non-detachable severe adhesion
<Evaluation Criteria of Adhesion Area>
0: No adhesion
1: The adhesion area is in a range of 1 to 24% of the abrasion site.
2: The adhesion area is in a range of 25 to 49% of the abrasion site.
3: The adhesion area is in a range of 50 to 74% of the abrasion site.
4: The adhesion area is in a range of 75 to 100% of the abrasion site.

### [Results]

The results of evaluating the degree of adhesion by score are shown in Table 8. In the non-treated group, all the mice were moderately and extensively adhered. In the group treated with SEPRAFILM, all the mice were slightly adhered, and SEPRAFILM disappeared. In the hydrogel, the adhesion preventing function is confirmed, and it is considered that the function is equivalent to or slightly superior to that of SEPRAFILM.

**[Table 8]**

| Treatment group | Degree of adhesion | Adhesion area |
|---|---|---|
| Sham group | 0 | 0 |
| Non-treated group | 3 | 3 |
| SEPRAFILM-treated group | 2.75 | 2.5 |
| Hydrogel-treated group | 2.25 | 2.25 |

### 6. In Situ Hydrogelation

Each of the polyrotaxane aqueous solution and the aqueous dispersion of the crosslinking agent EGDE was filled in a dual syringe having a volume ratio of 1 : 1. Both of the solutions were applied onto a glass slide while mixing in a static mixer, and the fluidity of the mixed solution was observed.

After 30 minutes from application, the mixed solution gelated and completely lost fluidity. From this result, it was found that when the polyrotaxane and the crosslinking agent were mixed, gelation occurred in a relatively short time under conditions of room temperature and water without requiring a catalyst.

## Claims

1. A polyrotaxane having a chemical structure in which an axial molecule represented by Formula (2) is threaded into a cyclodextrin ring in a compound represented by Formula (1): 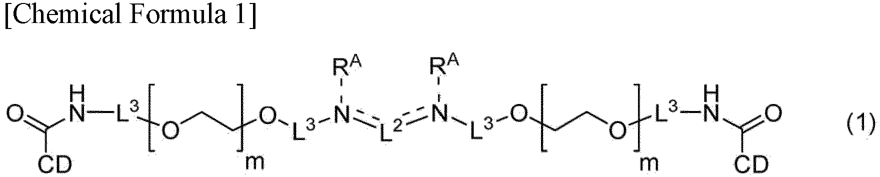
wherein CD is a cyclodextrin ring, L² is a divalent organic group, L³ is an ethylene group or a propylene group, R^{A} is a hydrogen atom or a C₁₋₆ alkyl group, and m is an integer of 0 to 35, provided that when L² and N are linked by a double bond, R^{A} is not present, 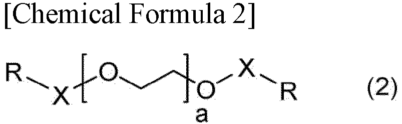
wherein R is a hydrocarbon group larger than an inner diameter of the cyclodextrin ring, X is a divalent organic group, and a is an integer of 100 to 1000.

2. A polyrotaxane having a chemical structure in which an axial molecule represented by Formula (2) is threaded into a cyclodextrin ring in a compound represented by Formula (1'): 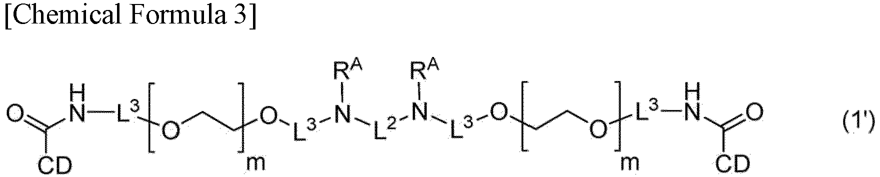
wherein CD is a cyclodextrin ring, L² is a divalent organic group, L³ is an ethylene group or a propylene group, R^{A} is a hydrogen atom or a C₁₋₆ alkyl group, and m is an integer of 0 to 35, 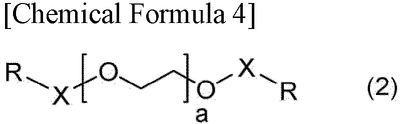
wherein R is a hydrocarbon group larger than an inner diameter of the cyclodextrin ring, X is a divalent organic group, and a is an integer of 100 to 1000.

3. The polyrotaxane according to claim 1 or 2, wherein the axial molecule is further threaded into a cyclodextrin ring in a compound represented by Formula (3): 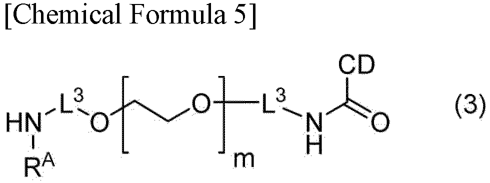 wherein CD is a cyclodextrin ring, L³ is an ethylene group or a propylene group, R^{A} is a hydrogen atom or a C₁₋₆ alkyl group, and m is an integer of 0 to 35.

4. The polyrotaxane according to claim 1 or 2, wherein the axial molecule is a compound represented by Formula (2a) or Formula (2b): 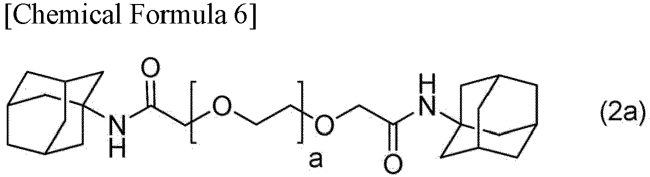
wherein a is an integer of 100 to 1000, 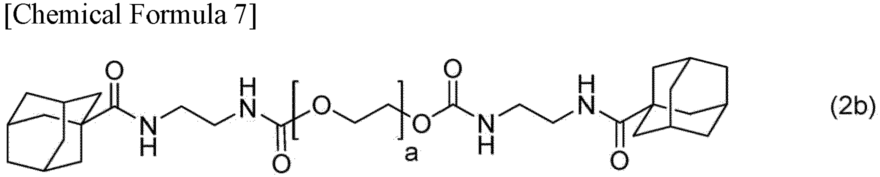
wherein a is an integer of 100 to 1000.

5. The polyrotaxane according to claim 1 or 2, wherein the compound represented by Formula (1) or Formula (1') is a compound represented by Formula (1a-1), (1a-2), or (1a-3): 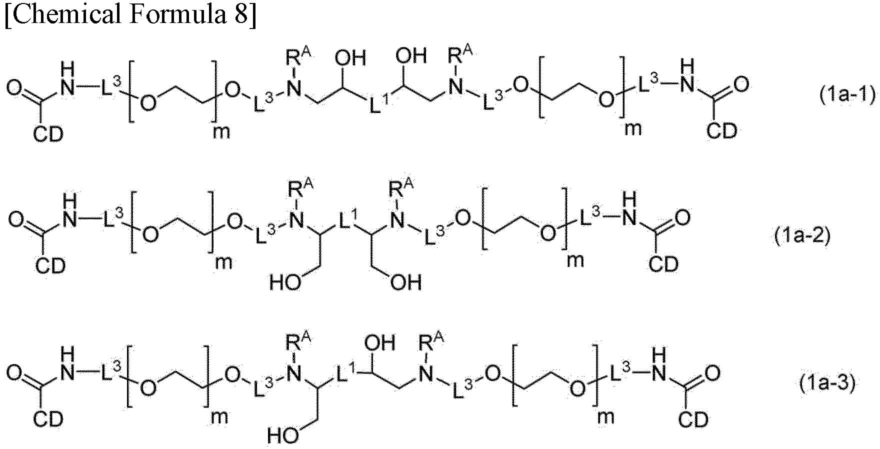 wherein CD is a cyclodextrin ring, L¹ is a divalent organic group, L³ is an ethylene group or a propylene group, R^{A} is a hydrogen atom or a C₁₋₆ alkyl group, and m is an integer of 0 to 35.

6. The polyrotaxane according to claim 1 or 2, wherein a transmittance of light at a wavelength of 600 nm is 40% or more.

7. A medical composition comprising the polyrotaxane according to claim 1 or 2.

8. The medical composition according to claim 7, which is in the form of a hydrogel.

9. The medical composition according to claim 7, which is a material for cell culture, a tissue adhesive, a substrate for tissue regeneration, or an adhesion preventing material.

10. The medical composition according to claim 8, which is a material for cell culture, a tissue adhesive, a substrate for tissue regeneration, or an adhesion preventing material.

11. A method for producing a polyrotaxane comprising a chemical structure in which an axial molecule represented by Formula (2) is threaded into a cyclodextrin ring in a compound represented by Formula (1): 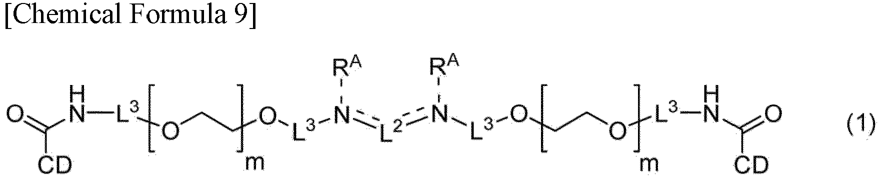
wherein CD is a cyclodextrin ring, L² is a divalent organic group, L³ is an ethylene group or a propylene group, R^{A} is a hydrogen atom or a C₁₋₆ alkyl group, and m is an integer of 0 to 35, provided that when L² and N are linked by a double bond, R^{A} is not present, 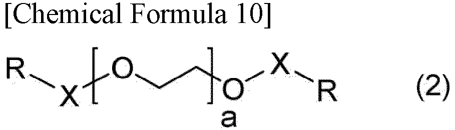
wherein R is a hydrocarbon group larger than an inner diameter of the cyclodextrin ring, X is a divalent organic group, and a is an integer of 100 to 1000,
the production method comprising a step of mixing a polyrotaxane (also referred to as "polyrotaxane A") having a chemical structure in which an axial molecule represented by Formula (2) is threaded into each cyclodextrin ring in one or a plurality of compounds represented by Formula (3): 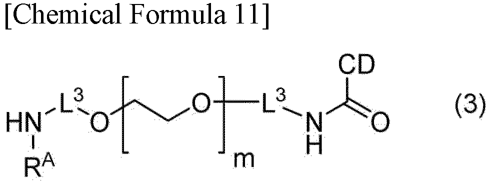
wherein CD is a cyclodextrin ring, L³ is an ethylene group or a propylene group, R^{A} is a hydrogen atom or a C₁₋₆ alkyl group, and m is an integer of 0 to 35, 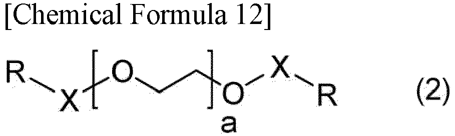
wherein R is a hydrocarbon group larger than an inner diameter of the cyclodextrin ring, X is a divalent organic group, and a is an integer of 100 to 1000,
with a crosslinking agent represented by Formula (4):
[Chemical Formula 13]
X¹-L¹-X¹ (4)
wherein L¹ is a divalent organic group, and X¹ is a group capable of reacting with an amino group.

12. A method for producing a polyrotaxane comprising a chemical structure in which an axial molecule represented by Formula (2) is threaded into a cyclodextrin ring in a compound represented by Formula (1'): 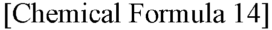 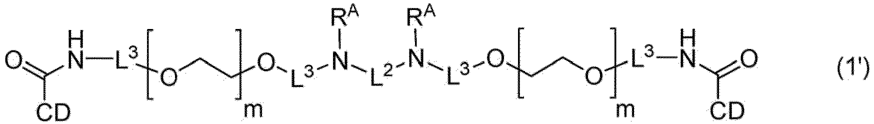
wherein CD is a cyclodextrin ring, L² is a divalent organic group, L³ is an ethylene group or a propylene group, R^{A} is a hydrogen atom or a C₁₋₆ alkyl group, and m is an integer of 0 to 35, 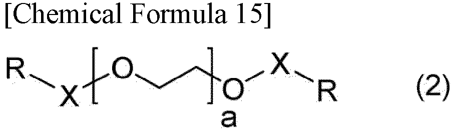
wherein R is a hydrocarbon group larger than an inner diameter of the cyclodextrin ring, X is a divalent organic group, and a is an integer of 100 to 1000,
the production method comprising a step of mixing a polyrotaxane (also referred to as "polyrotaxane A") having a chemical structure in which an axial molecule represented by Formula (2) is threaded into each cyclodextrin ring in one or a plurality of compounds represented by Formula (3): 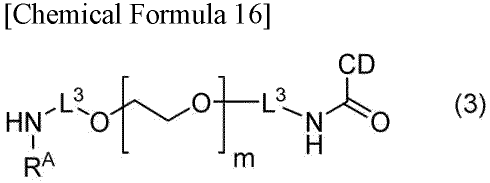
wherein CD is a cyclodextrin ring, L³ is an ethylene group or a propylene group, R^{A} is a hydrogen atom or a C₁₋₆ alkyl group, and m is an integer of 0 to 35, 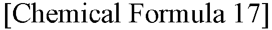 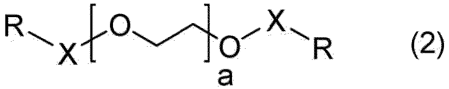
wherein R is a hydrocarbon group larger than an inner diameter of the cyclodextrin ring, X is a divalent organic group, and a is an integer of 100 to 1000,
with a crosslinking agent represented by Formula (4):
[Chemical Formula 18]
X¹-L¹-X¹ (4)
wherein L¹ is a divalent organic group, and X¹ is a group capable of reacting with an amino group.

13. The production method according to claim 11 or 12, wherein in the polyrotaxane having a chemical structure in which an axial molecule represented by Formula (2) is threaded into a cyclodextrin ring in a compound represented by Formula (1) or Formula (1'), the axial molecule is further threaded into a cyclodextrin ring in a compound represented by Formula (3): 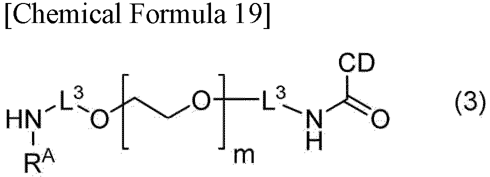 wherein CD is a cyclodextrin ring, L³ is an ethylene group or a propylene group, R^{A} is a hydrogen atom or a C₁₋₆ alkyl group, and m is an integer of 0 to 35.

14. The production method according to claim 11 or 12, wherein the step of mixing the polyrotaxane A with the crosslinking agent represented by Formula (4) is performed in the absence of a catalyst.

15. A kit comprising:
a polyrotaxane having a chemical structure in which an axial molecule represented by Formula (2) is threaded into each cyclodextrin ring in one or a plurality of compounds represented by Formula (3); and 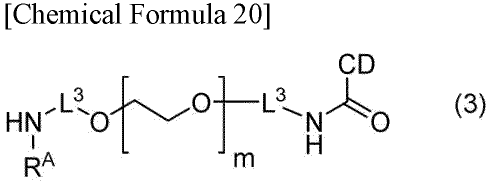
wherein CD is a cyclodextrin ring, L³ is an ethylene group or a propylene group, R^{A} is a hydrogen atom or a C₁₋₆ alkyl group, and m is an integer of 0 to 35, 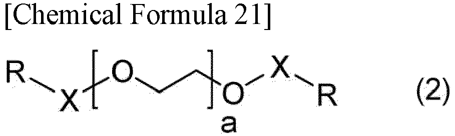
where R is a hydrocarbon group larger than an inner diameter of the cyclodextrin ring, X is a divalent organic group, and a is an integer of 100 to 1000,
a crosslinking agent represented by Formula (4):
[Chemical Formula 22]
X¹-L¹-X¹ (4)
wherein L¹ is a divalent organic group, and X¹ is a group capable of reacting with an amino group.

16. The method for producing a polyrotaxane according to claim 11 or 12, wherein the compound represented by Formula (1) or Formula (1') is a compound represented by Formula (1a-1), (1a-2), or (1a-3): 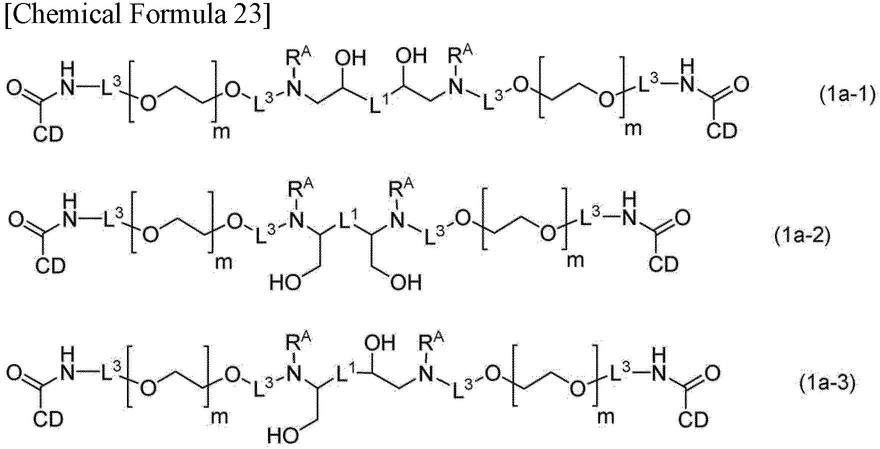 wherein CD is a cyclodextrin ring, L¹ is a divalent organic group, L³ is an ethylene group or a propylene group, R^{A} is a hydrogen atom or a C₁₋₆ alkyl group, and m is an integer of 0 to 35.

17. A polyrotaxane having a chemical structure in which an axial molecule represented by Formula (2) is threaded into each cyclodextrin ring in one or a plurality of compounds represented by Formula (3): 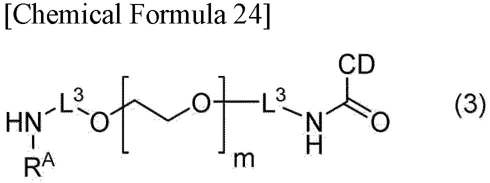
wherein CD is a cyclodextrin ring, L³ is an ethylene group or a propylene group, R^{A} is a hydrogen atom or a C₁₋₆ alkyl group, and m is an integer of 0 to 35, 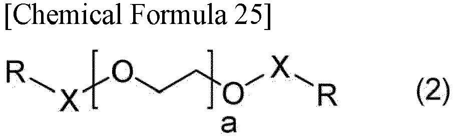
where R is a hydrocarbon group larger than an inner diameter of the cyclodextrin ring, X is a divalent organic group, and a is an integer of 100 to 1000.
